(19) Europäisches Patentamt
European Patent Office
Office européen des brevets

(11) **EP 4 067 488 A1**

(12) **EUROPEAN PATENT APPLICATION**
published in accordance with Art. 153(4) EPC

(43) Date of publication:
**05.10.2022 Bulletin 2022/40**

(21) Application number: **20894793.7**

(22) Date of filing: **27.11.2020**

(51) International Patent Classification (IPC):
**C12N 15/09** (2006.01)     **C12Q 1/686** (2018.01)
**C12Q 1/6869** (2018.01)     **C12Q 1/6886** (2018.01)

(52) Cooperative Patent Classification (CPC):
**C12N 15/09; C12Q 1/686; C12Q 1/6869; C12Q 1/6886**

(86) International application number:
**PCT/JP2020/044181**

(87) International publication number:
**WO 2021/107081 (03.06.2021 Gazette 2021/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.11.2019 JP 2019214647**

(71) Applicant: **Keio University**
**Tokyo, 108-8345 (JP)**

(72) Inventors:
• **KANAI, Yae**
**Tokyo 160-8582 (JP)**

• **ARAI, Eri**
**Tokyo 160-8582 (JP)**
• **FUJIMOTO, Mao**
**Tokyo 160-8582 (JP)**
• **YOTANI, Takuya**
**Tokyo 103-0027 (JP)**
• **YAMADA, Yuriko**
**Tokyo 103-0027 (JP)**

(74) Representative: **Schiener, Jens**
**Wächtershäuser & Hartz**
**Patentanwaltspartnerschaft mbB**
**Weinstraße 8**
**80333 München (DE)**

(54) **UPPER TRACT UROTHELIAL CARCINOMA IDENTIFICATION METHOD**

(57)     Provided is a method for diagnosing upper urinary tract urothelial carcinoma with low invasiveness and high reliability. A method for identifying a cell or tissue having upper urinary tract urothelial carcinoma, comprising: detecting the DNA methylation level of at least one of specific CpG sites in genomic DNA derived from an upper urinary tract urothelial cell or a tissue containing the upper urinary tract urothelial cell; and determining whether the cell or tissue containing such a cell has upper urinary tract urothelial carcinoma on the basis of the detected DNA methylation level.

EP 4 067 488 A1

**Description**

Technical Field

The present invention relates to an upper urinary tract urothelial carcinoma identification method.

Background Art

Urothelial carcinoma (UC) is a carcinoma that occurs in the upper urinary tract (that is, the renal pelvis and the ureter) and the bladder, being the second most common urologic malignant tumor next to prostate carcinoma. UC with the highest incidence is urinary bladder urothelial carcinoma (BUC). Upper urinary tract urothelial carcinoma (UTUC) is relatively rare, but generally exhibits poor prognosis.

The first symptom in UC patients is typically macroscopic or microscopic hematuria. Patients with hematuria are normally subjected to voided urine cytology, and positive patients are further subjected to cystoscopy. If no tumor was found in the bladder, the patient is suspected to have UTUC.

In conventional test methods for UTUC, ureteral catheterization has been performed for retrograde ureteropyelography, ureteroscopy, and selective urine cytology sampling. However, ureteral catheterization is highly invasive because a catheter is inserted into a narrow space, the bladder, and sometimes induces complications including urinary tract infections and ureteral injury or transection. The selective urine cytology does not necessarily provide sufficient sensitivity and specificity because of the break and denaturation of carcinoma cells detached off. Accordingly, ureteral catheterization tends to be avoided in recent years. MRI is non-invasive, but is incapable of depicting calyces and the like in a sufficient manner because of the low resolution. CT-urography is recently recommended as a test method for UTUC. However, the CT-urography disadvantageously causes higher exposure doses than retrograde ureteropyelography, being a contraindication to contrast media allergy and renal impairment cases. Moreover, diagnostic imaging through CT-urography, MRI, or the like is incapable of detecting flat intraepithelial carcinoma without urothelial hyperplasia. It is desired to develop a diagnostic method for UTUC with low invasiveness and high reliability.

In recent years, it has become clear that abnormal methylation of DNA is deeply involved in canceration, and has been attracting attention. Abnormal DNA methylation of CpG islands in some gene promoter regions is known as a characteristic epigenetic abnormality in carcinoma cells. A CpG island is a region in which a dibasic sequence of cytosine (C)-guanine (G) via a phosphodiester bond (p) frequently appears. Abnormal DNA methylation of CpG islands is involved in carcinogenesis through inactivation of tumor suppressor genes and the like.

Regarding urothelial carcinoma, the group of the present inventors and other groups have conducted research to report that non-cancerous urothelium exhibits a DNA methylation profile different from that of UC (Non Patent Literatures 1 to 6), and it has been suggested that the change in DNA methylation is available for diagnosis of UC (Non Patent Literature 7). Although these previous reports suggest the possibility of risk determination for UC based on abnormal DNA methylation, however, they do not suggest that it is possible to establish a diagnostic method for upper urinary tract urothelial carcinoma as a substitute for the conventional ureteral catheterization and CT-urography on the basis of abnormal DNA methylation.

On the other hand, differences in DNA methylation profiles and gene mutation between UTUC and BUC have been recently reported (Non Patent Literatures 8 and 9). At least part of the difference in DNA methylation profiles between UTUC and BUC may be due to the difference in gene mutation therebetween.

As a method for analyzing methylated DNA, a method utilizing a bisulfite method has already been established and is widely used. The Methylation-Specific PCR (MSP) method, Combined Bisulfite Restriction Analysis (COBRA) method, and BAC array-based methylated CpG island amplification method (BAMCA method) are often used as methylated DNA analysis methods based upon the bisulfite method. Patent Literature 1 discloses a method for detecting the risk of poor prognosis for renal cell carcinoma by detecting the methylation level of the CpG sites of a specific gene by a bead array method, mass spectrometry (MassARRAY method), pyrosequencing, methylation-sensitive high resolution melting curve analysis, quantitative PCR, direct sequencing of bisulfite-treated products, COBRA method and the like. Patent Literatures 2 and 3 disclose methods for determining the prognosis of carcinoma by detecting the methylation level of the CpG sites of a specific gene based on the difference in retention time by ion exchange chromatography.

Citation List

Patent Literature

Patent Literature 1: WO 2013/168644

Patent Literature 2: WO 2015/129916
Patent Literature 3: WO 2017/038983

Non Patent Literature

Non Patent Literature 1: Carcinogenesis, 2011, 32:462-9
Non Patent Literature 2: Cancer Sci, 2010, 101:231-40
Non Patent Literature 3: J Urology, 2005, 173:243-6
Non Patent Literature 4: Curr Urol Rep, 2018, 19:102
Non Patent Literature 5: Epigenomics, 2016, 8:1415-1428
Non Patent Literature 6: Nature, 2014, 507:315-22
Non Patent Literature 7: Carcinogenesis, 2019, bgz112
Non Patent Literature 8: Int J Mol Sci, 2019, 20:E2657
Non Patent Literature 9: Eur Urol, 2017, 72:641-649

Summary of Invention

Technical Problem

Diagnostic methods for upper urinary tract urothelial carcinoma (UTUC) with low invasiveness and high reliability are desired. The present invention provides a method for diagnosing UTUC on the basis of the methylation level of DNA.

Solution to Problem

The present inventors identified CpG which shows DNA methylation profiles different between upper urinary tract urothelial carcinoma and non-cancerous upper urinary tract urothelium. Moreover, the present inventors found that UTUC and urinary bladder urothelial carcinoma (BUC) can be distinguished from each other on the basis of such DNA methylation profiles of CpG.

Therefore, the present invention provides the following.

[1] A method for identifying a cell or tissue having upper urinary tract urothelial carcinoma, comprising:

detecting a DNA methylation level of at least one CpG site in genomic DNA derived from an upper urinary tract urothelial cell or a tissue containing the upper urinary tract urothelial cell, wherein the at least one CpG site is selected from the group consisting of position 158,799,748, position 158,799,765, position 158,799,775, position 158,799,777, and position 158,799,789 on chromosome 7; position 111,813,685, position 111,813,690, and position 111,813,698 on chromosome 1; position 240,375,464 on chromosome 1; position 28,956,374, position 28,956,381, position 28,956,384, and position 28,956,387 on chromosome 6; position 91,182,528 and position 91,182,534 on chromosome 1; position 119,535,921, position 119,535,925, position 119,535,928, and position 119,535,937 on chromosome 1; position 4,850,072, position 4,850,075, and position 4,850,090 on chromosome 7; position 31,846,849, position 31,846,844, position 31,846,839, and position 31,846,833 on chromosome 11; and position 106,187,192 and position 106,187,210 on chromosome 14; and

determining whether the cell or tissue has upper urinary tract urothelial carcinoma on the basis of the detected DNA methylation level.

[2] The method according to [1], wherein the detection of the DNA methylation level comprises detecting the DNA methylation level of the at least one CpG site using the genomic DNA treated with bisulfite.

[3] The method according to [2], wherein the detection of the DNA methylation level is performed using a pyrosequencing method, mass spectrometry, a bead array method or ion exchange chromatography.

[4] The method according to any one of [1] to [3], wherein the genomic DNA is DNA derived from an upper urinary tract urothelial cell contained in urine.

[5] A primer or probe for identifying upper urinary tract urothelial carcinoma, which has a chain length of at least 12 bases, and is hybridized to at least one CpG site selected from the group consisting of position 158,799,748, position 158,799,765, position 158,799,775, position 158,799,777, and position 158,799,789 on chromosome 7; position 111,813,685, position 111,813,690, and position 111,813,698 on chromosome 1; position 240,375,464 on chromosome 1; position 28,956,374, position 28,956,381, position 28,956,384, and position 28,956,387 on chromosome 6; position 91,182,528 and position 91,182,534 on chromosome 1; position 119,535,921, position 119,535,925, position 119,535,928, and position 119,535,937 on chromosome 1; position 4,850,072, position 4,850,075, and position 4,850,090 on chromosome 7; position 31,846,849, position 31,846,844, position 31,846,839, and position 31,846,833 on chromosome 11; and position 106,187,192 and position 106,187,210 on chromosome 14 after being treated with bisulfite.

[6] The primer or probe according to [5], which is hybridized to a bisulfite-treated product of DNA containing a nucleotide sequence set forth in any of SEQ ID NOs: 31 to 40.

[7] The primer or probe according to [5] or [6], which is selected from the group consisting of a polynucleotide consisting of a nucleotide sequence set forth in any of SEQ ID NOs: 1 to 30 and a complementary strand thereof.

[8] The primer or probe according to [7], which is

a primer set consisting of a combination of the polynucleotides set forth in SEQ ID NOs: 1 and 2, or a combination of complementary strands thereof,

a primer set consisting of a combination of the polynucleotides set forth in SEQ ID NOs: 3 and 4, or a combination of complementary strands thereof,

a primer set consisting of a combination of the polynucleotides set forth in SEQ ID NOs: 5 and 6, or a combination of complementary strands thereof,

a primer set consisting of a combination of the polynucleotides set forth in SEQ ID NOs: 7 and 8, or a combination of complementary strands thereof,

a primer set consisting of a combination of the polynucleotides set forth in SEQ ID NOs: 9 and 10, or a combination of complementary strands thereof,

a primer set consisting of a combination of the polynucleotides set forth in SEQ ID NOs: 11 and 12, or a combination of complementary strands thereof,

a primer set consisting of a combination of the polynucleotides set forth in SEQ ID NOs: 13 and 14, or a combination of complementary strands thereof,

a primer set consisting of a combination of the polynucleotides set forth in SEQ ID NOs: 15 and 16, or a combination of complementary strands thereof,

a primer set consisting of a combination of the polynucleotides set forth in SEQ ID NOs: 17 and 18, or a combination of complementary strands thereof, or

a primer set consisting of a combination of the polynucleotides set forth in SEQ ID NOs: 19 and 20, or a combination of complementary strands thereof.

[9] The primer or probe according to [7], which is a probe consisting of a polynucleotide consisting of a nucleotide sequence set forth in any of SEQ ID NOs: 21 to 30 or a complementary strand thereof.

[10] A method for identifying upper urinary tract urothelial carcinoma in a subject, comprising:

detecting a DNA methylation level of at least one CpG site in genomic DNA derived from an upper urinary tract urothelial cell or a tissue containing the upper urinary tract urothelial cell of a subject, wherein the at least one CpG site is selected from the group consisting of position 158,799,748, position 158,799,765, position 158,799,775, position 158,799,777, and position 158,799,789 on chromosome 7; position 111,813,685, position 111,813,690, and position 111,813,698 on chromosome 1; position 240,375,464 on chromosome 1; position 28,956,374, position 28,956,381, position 28,956,384, and position 28,956,387 on chromosome 6; position 91,182,528 and position 91,182,534 on chromosome 1; position 119,535,921, position 119,535,925, position 119,535,928, and position 119,535,937 on chromosome 1; position 4,850,072, position 4,850,075, and position 4,850,090 on chromosome 7; position 31,846,849, position 31,846,844, position 31,846,839, and position 31,846,833 on chromosome 11; and position 106,187,192 and position 106,187,210 on chromosome 14; and determining whether the subject has upper urinary tract urothelial carcinoma on the basis of the detected DNA methylation level.

[11] The method according to [10], wherein the detection of the DNA methylation level comprises detecting the DNA methylation level of the at least one CpG site using the genomic DNA treated with bisulfite.

[12] The method according to [11], wherein the detection of the DNA methylation level is performed using a pyrosequencing method, mass spectrometry, a bead array method or ion exchange chromatography.

[13] The method according to any one of [10] to [12], wherein the genomic DNA is DNA derived from an upper urinary tract urothelial cell contained in urine.

Advantageous Effects of Invention

According to the present invention, tissues having UTUC can be identified with low invasiveness and high sensitivity and specificity. The present invention enables accurate diagnosis of UTUC with reduced physical burdens on a patient in UTUC test. Moreover, the present invention contributes to the improvement of survival rates of UTUC patients by enabling UTUC test for patients to which it is difficult to apply the conventional ureteral catheterization and CT-urography.

Brief Description of Drawings

[Figure 1] Principal component analysis of results of Infinium assay for 473,228 CpG sites. Circular symbols: normal upper urinary tract urothelial tissue samples (C) (n=26), triangular symbols: non-cancerous upper urinary tract urothelial tissue samples from UTUC patients (N) (n=31), cross symbols: UTUC tissue samples (UTUC) (n=31).
[Figure 2] Unsupervised hierarchical clustering using results of Infinium assay for C samples, N samples and UTUC samples.
[Figure 3] Quantitative values of the DNA methylation levels of 10 CpG sites in pyrosequencing. The CpG sites are designated by probe ID in Infinium assay.
[Figure 4] ROC curves on sensitivity and specificity to distinguish between UTUC samples (n=30) and N samples (n=31) for 37 markers.
[Figure 5] The continuation from Figure 4.
[Figure 6] An exemplary chromatogram for DNA derived from a urine sample from a UTUC patient. Shown together with chromatograms for a negative control (unmethylated DNA) and a positive control (methylated DNA).
[Figure 7] Shown are the original peak in the chromatogram shown in Figure 6, which was obtained from a urine sample from a UTUC patient, and peaks estimated as the constituent components.

Description of Embodiments

In the present description, "upper urinary tract" refers to the upper region of the urinary tract ranging from the renal pelvis to the urethra, that is, the renal pelvis and the ureter, without including more distal parts of the urinary tract, that is, the bladder and the urethra. In the present description, "upper urinary tract urothelial carcinoma (UTUC)" refers to a carcinoma that occurs in the epithelial cells of the upper urinary tract, without including carcinomas that occur in a part of the urinary tract distal from the ureter such as urinary bladder urothelial carcinoma (BUC). "Upper urinary tract urothelial carcinoma" in the present description includes primary carcinoma and carcinoma resulting from the metastasis of primary upper urinary tract carcinoma within the upper urinary tract.
In the present description, the "subject" is a human, and examples thereof include a person who needs identification of UTUC, and more specific examples thereof include a medical check examinee and a patient suspected of being affected by UTUC.
In the present description, the "CpG site" means a site where cytosine (C) and guanine (G) are bonded by a phosphodiester bond (p) in DNA. A region where CpG sites appear with high frequency is called a CpG island. CpG sites contained in CpG islands often involve abnormal DNA methylation in association with carcinoma. In the present description, the "CpG site", unless otherwise defined, preferably means a CpG site contained in a CpG island, and more preferably means a CpG site located in the vicinity of the gene.
In the present description, "DNA methylation" means a state in which the carbon at position 5 of cytosine is methylated in DNA. Moreover, in the present description, the "DNA methylation level" of a CpG site means the proportion of methylated DNA at the CpG site. In the present description, a high or low DNA methylation level means that the proportion of methylated DNA is high or low, respectively.
In the present description, "at least 90% identity" with respect to an amino acid sequence and a nucleotide sequence refers to 90% or more identity, preferably 93% or more identity, more preferably 95% or more identity, further preferably 96% or more identity, furthermore preferably 97% or more identity, furthermore preferably 98% or more identity, furthermore preferably 99% or more identity.
As demonstrated later in Examples, the present inventors revealed that the DNA methylation levels of CpG sites located at specific loci on the chromosome differ between tissue without UTUC derived from a non-UTUC or UTUC patient and tissue with UTUC derived from a UTUC patient. The DNA methylation levels of those CpG sites were found to differ between UTUC and BUC. Accordingly, the DNA methylation levels of the CpG sites can serve as markers for identification of UTUC.
Table 1 shows CpG sites available for identification of UTUC in the present invention. In the present description, the position of a CpG site on the chromosome is expressed based on the position on the NCBI database Genome Build 37, which is a human reference genome sequence.

[Table 1]

List of CpG sites as UTUC identification markers

| Marker group | Chromosome number | Position on the chromosome | Gene symbol* | Change in methylation associated with UTUC |
|---|---|---|---|---|
| 1 | 1 | 91,182,528<br>91,182,534 | BARHL2 | UTUC > N |
| 2 | 1 | 111,813,685<br>111,813,690<br>111,813,698 | NA | UTUC > N |
| 3 | 1 | 240,375,464 | FMN2 | UTUC < N |
| 4 | 11 | 31,846,849<br>31,846,844<br>31,846,839<br>31,846,833 | NA | UTUC > N |
| 5 | 7 | 4,850,072<br>4,850,075<br>4,850,090 | RADIL | UTUC < N |
| 6 | 7 | 158,799,748<br>158,799,765<br>158,799,775<br>158,799,777<br>158,799,789 | LOC154822 | UTUC < N |
| 7 | 14 | 106,187,192<br>106,187,210 | NA | UTUC < N |
| 8 | 6 | 28,956,374<br>28,956,381<br>28,956,384<br>28,956,387 | NA | UTUC > N |
| 9 | 1 | 119,535,921<br>119,535,925<br>119,535,928<br>119,535,937 | NA | UTUC > N |

\* NA: not annotated (disposed in intergenic region)

In the present invention, the DNA methylation levels of the CpG sites shown in Table 1 can be used as markers for identification of UTUC.

The CpG sites shown in Table 1 are classified into marker groups 1 to 9 in the list. Each marker group is composed of CpG sites close to each other, and the CpG sites close to each other are together subjected to DNA methylation or unmethylation in association with UTUC. Accordingly, in the present invention, a statistic (for example, a total value, a mean, but not limited thereto) of the DNA methylation levels of CpG sites included in each marker group may be used as a marker for identification of UTUC.

Thus, the present invention provides a method for identifying a cell or tissue having UTUC, comprising:

detecting the DNA methylation level of at least one CpG site in genomic DNA derived from an upper urinary tract urothelial cell of a subject or a tissue containing an upper urinary tract urothelial cell of a subject, wherein the at least one CpG site is selected from the group consisting of the CpG sites shown above in Table 1; and determining whether the cell or tissue has UTUC on the basis of the detected DNA methylation level.

Further, the present invention provides a method for identifying UTUC in a subject, comprising:

detecting the DNA methylation level of at least one CpG site in genomic DNA derived from an upper urinary tract urothelial cell of a subject or a tissue containing an upper urinary tract urothelial cell of a subject, wherein the at least one CpG site is selected from the group consisting of the CpG sites shown above in Table 1; and determining whether the subject has UTUC on the basis of the detected DNA methylation level.

Furthermore, the present invention provides a method for acquiring data for use in identification of UTUC in a subject or data for use in identification of a cell or tissue having UTUC, comprising:

detecting the DNA methylation level of at least one CpG site in genomic DNA derived from an upper urinary tract urothelial cell of a subject or a tissue containing an upper urinary tract urothelial cell of a subject, wherein the at least one CpG site is selected from the group consisting of the CpG sites shown above in Table 1; and acquiring data for determining whether the subject has UTUC or data for determining whether the cell or tissue has UTUC on the basis of the detected DNA methylation level.

Although the DNA methylation level of any one of the CpG sites shown in Table 1 can be detected in the method of the present invention, the DNA methylation levels of any two or more CpG sites may be detected. If the DNA methylation levels of two or more CpG sites are detected, a combination of CpG sites belonging to the same marker group shown in Table 1 may be used, and a combination of CpG sites belonging to different marker groups may be used. Alternatively, a statistic (for example, a total value, a mean, but not limited thereto) of the DNA methylation levels of CpG sites included in any of the marker groups shown in Table 1 may be detected. Statistics of the DNA methylation levels derived from different marker groups may be used in combination. One or more statistics of the DNA methylation levels and the DNA methylation level of a CpG site belonging to a marker group different from the marker groups for the statistics may be used in combination.

Examples of the genomic DNA derived from an upper urinary tract urothelial cell or a tissue containing the upper urinary tract urothelial cell to be used in the method of the present invention include genomic DNA prepared from an upper urinary tract urothelial cell or a tissue containing the upper urinary tract urothelial cell, and DNA derived from an upper urinary tract urothelial cell in urine. Examples of the urothelial cells or tissues containing the same include fresh upper urinary tract urothelial tissue collected in surgery or biopsy, frozen upper urinary tract urothelial tissue frozen after collection, upper urinary tract urothelial tissue that has been fixed in formalin and embedded in paraffin after collection, and upper urinary tract urothelial cells concentrated from urine. Among these, frozen upper urinary tract urothelial tissue is preferable from the viewpoint of suppressing the degradation of genomic DNA in the tissue and more efficiently detecting the DNA methylation level. From the viewpoints of non-invasiveness and easiness in collection, DNA derived from an upper urinary tract urothelial cell contained in voided urine is preferable.

The method for preparing the sample DNA from the above-mentioned upper urinary tract urothelial cell or tissue containing an upper urinary tract urothelial cell is not particularly limited, and a known method can be appropriately selected and used. Known methods for preparing DNA include the phenol-chloroform method, or a DNA extraction method using a commercially available DNA extraction kit, for example, QIAamp DNA Mini kit (manufactured by Qiagen), Clean Columns (manufactured by NexTec), AquaPure (manufactured by Bio-Rad), ZR Plant/Seed DNA Kit (manufactured by Zymo Research), prepGEM (manufactured by ZyGEM), BuccalQuick (manufactured by TrimGen), and the like. The urothelial cell-derived DNA or cell-free DNA in urine can also be prepared using a commercially available kit for purifying cell-derived DNA or cell-free DNA in urine.

Preferably, the prepared genomic DNA is treated with bisulfite. The method for the bisulfite treatment of DNA is not particularly limited, and a known method can be appropriately selected and used. Examples of known methods for the bisulfite treatment include methods using a commercially available kit such as an EZ DNA Methylation-Gold (TM) Kit (manufactured by Zymo Research), an EpiTect Bisulfite Kit (manufactured by Qiagen), MethylEasy (manufactured by Human Genetics Signatures Pty), Cells-to-CpG Bisulfite Conversion Kit (manufactured by Applied Biosystems), and CpGenome Turbo Bisulfite Modification Kit (manufactured by MERCK MILLIPORE).

Furthermore, the bisulfite-treated DNA may be amplified. The method of amplification is not particularly limited, but PCR is preferably used. As for the method and conditions of amplification, known methods and conditions can be appropriately selected and used according to the sequence, length, amount, and the like of the DNA to be amplified.

If the bisulfite-treated DNA is amplified by PCR or the like, it is only required that DNA containing at least one of the CpG sites shown in Table 1 is amplified. Preferable examples of the region to be amplified include one or more selected from the group consisting of DNA regions including a nucleotide sequence set forth in any of SEQ ID NOs: 31 to 40. The chain length of the PCR amplification products can be appropriately selected while considering factors such as shortening the PCR amplification time, shortening the time for detecting methylation levels, and the precision of detection of methylation levels. For example, the chain length of the PCR amplification products is preferably 500 bp or less, more preferably 300 bp or less, and further preferably 100 bp or less; on the other hand, the lower limit of the chain length of the PCR amplification products is 30 to 40 bp, which is the chain length of the PCR amplification products when using a primer of around 15mer which can avoid non-specific hybridization in PCR. Otherwise, it is preferable to design the primer to have a rich content of CpG sites shown in Table 1 in the PCR amplification products. For example, it is preferable that the cytosine of the CpG site be contained in 2% or more, more preferably 5% or more, with respect to the chain length of the PCR amplification products.

In the method of the present invention, the method for detecting the DNA methylation level of a CpG site should be a method that can quantify the DNA methylation level at a given CpG site, and a known method can be appropriately selected. Examples of such known methods include the first to eighth methods shown below.

The first method is a method which utilizes a single-base extension reaction using a probe constructed so as to have a base complementary to methylated cytosine or unmethylated cytosine at the 3'-terminus, to quantify the methylation of DNA at a CpG site. For example, the first method is a method based on the following principles. First, a bisulfite treatment is performed on the genomic DNA. As a result of this bisulfite treatment, unmethylated cytosine residues are converted to uracil, but methylated cytosine residues are not converted (see Clark SJ et al., Nucleic Acids Res, 1994, 22, 2990-2997). Next, the whole genome is amplified using the genomic DNA thus treated with bisulfite as a template, and enzymatic fragmentation (usually, fragmentation into about 300 to 600 bp) is performed to dissociate it into a single strand.

In the first method, a probe, which hybridizes to genomic DNA converted by bisulfite treatment, and in which the base at the 3'-terminus of the probe is a base complementary to cytosine at the CpG site, is prepared. That is, when the CpG site is methylated, the base at the 3'-terminus of the probe is guanine, meanwhile when the CpG site is not methylated, the base at the 3'-terminus of the probe is adenine.

Then, these two types of probes different only in the base at the 3'-terminus complementary to the CpG site are hybridized with the single-stranded DNA fragment, and a single-base extension reaction is performed in the presence of a fluorescently labeled base. As a result, when the CpG site of the single-stranded fragment is methylated, a fluorescently labeled base is incorporated into the probe in which the 3'-terminal base is guanine (probe for methylation detection) by a single-base extension reaction, but no fluorescently labeled base is incorporated into the probe in which the 3'-terminal base is adenine (probe for unmethylation detection) as no single-base extension reaction occurs due to the mismatch of the 3'-terminal base. On the other hand, when the CpG site of the single-stranded fragment is not methylated, a fluorescently labeled base is incorporated into the probe for unmethylation detection, but no fluorescently labeled base is incorporated into the probe for methylation detection. Therefore, the DNA methylation level can be calculated from the intensity of fluorescence emitted by the probe for methylation detection and/or the probe for unmethylation detection.

In addition, in this first method, as another aspect, a probe, which hybridizes to genomic DNA converted by bisulfite treatment, and in which the 3'-terminal base of the probe is a base complementary to guanine at the CpG site, may be used instead of the probe for methylation detection and the probe for unmethylation detection. Then, this probe is hybridized to the single-stranded DNA fragment, and a single-base extension reaction is performed in the presence of guanine labeled with a fluorescent substance and/or adenine labeled with a fluorescent dye different from the fluorescent substance. As a result, when the CpG site is methylated, fluorescently labeled guanine is incorporated into the probe, whereas when the CpG site is not methylated, fluorescently labeled adenine is incorporated into the probe. Therefore, the DNA methylation level can be calculated from the intensity of the fluorescence emitted by each fluorescent substance incorporated into the probe.

A preferable example of this first method is, for example, a bead array method (for example, Infinium assay provided by Illumina, Inc.).

The second method is a method for quantifying methylated DNA by mass spectrometry. For example, the second method is a method based on the following principles. First, a bisulfite treatment is performed on the genomic DNA. Next, using the bisulfite-treated genomic DNA as a template, DNA containing at least one of the CpG sites is amplified with a primer to which a T7 promoter is added. Next, the amplified DNA is transcribed into RNA, and a base-specific cleavage reaction is performed with RNase. Then, the product of this cleavage reaction is subjected to a mass spectrometer to measure the mass.

Then, the mass derived from the methylated cytosine residue (mass of cytosine) obtained by mass measurement is compared to the mass derived from the unmethylated cytosine residue (mass of uracil) to calculate the DNA methylation level at the CpG site.

A preferable example of this second method is, for example, a DNA methylation analysis method using a mass spectrometer (for example, MassARRAY (R), see Jurinke C et al., Mutat Res, 2005, 573, 83-95).

In MassARRAY (registered trademark), bisulfite-treated DNA containing the CpG sites is amplified, transcribed into RNA, and then specifically cleaved at the uracil site by RNAase. As a result, RNA fragments having different lengths are produced according to the presence or absence of methylation of DNA. The obtained RNA fragments are subjected to mass spectrometry, which allows to separate and detect the CpG-methylated fragments from the unmethylated fragments according to the difference in molecular weight. The primer for amplifying DNA containing the CpG sites can be designed using EpiDesigner (manufactured by SEQUENOM, primer design software for MassARRAY) and the like. For the mass spectrometry of the RNA fragments, MALDI-TOF MAS (for example, MassARRAY Analyzer 4 manufactured by SEQUENOM), which can detect the difference in mass of a single base, is used. The methylation level of DNA is calculated from the mass ratio between the RNA fragments derived from methylated DNA and the RNA fragments derived from unmethylated DNA.

The third method is a method based on the following principles. First, a bisulfite treatment is performed on the genomic DNA. This bisulfite treatment converts unmethylated cytosine residues to uracil, but uracil is shown as thymine in the following extension reaction (sequence reaction). Next, using the genomic DNA thus treated with bisulfite as a template, DNA containing at least one of the CpG sites is amplified. Then, the amplified DNA is dissociated into single strands. Next, only one strand of the dissociated single-stranded DNA is separated. Then, an extension reaction is performed on each base from the vicinity of the base of the CpG site, pyrophosphoric acid generated at that time is enzymatically made luminescent, and the intensity of luminescence is measured. The intensity of luminescence derived from the methylated cytosine residues thus obtained (luminescence intensity of cytosine) is compared to the intensity of luminescence derived from the unmethylated cytosine residues (luminescence intensity of thymine), and the DNA methylation level (%) at the CpG site is calculated by, for example, the following expression:

```
DNA methylation level (%) = luminescence intensity

of cytosine x 100 / (luminescence intensity of cytosine +

luminescence intensity of thymine).
```

Examples of the third method include the Pyrosequencing method (registered trademark) (see Anal. Biochem. (2000) 10: 103-110) and the like.

The fourth method is a method based on the following principles. First, a bisulfite treatment is performed on the genomic DNA. Next, in a reaction system containing an intercalator which emits fluorescence when inserted between DNA duplexes, the nucleotides containing at least one of the CpG sites are amplified using the bisulfite-treated genomic DNA as a template. Then, the temperature of the reaction system is changed to detect the change in the intensity of the fluorescence emitted by the intercalator. The melting curve of the nucleotides containing at least one of the CpG sites is compared to the melting curve of the amplification product using the methylated/unmethylated control specimen as a template to calculate the DNA methylation level at the CpG site.

An example of the fourth method is the methylation-sensitive high resolution melting (MS-HRM, see Wojdacz TK et al., Nat Protoc., 2008, 3, 1903-1908).

The fifth method is a method based on the following principles. First, a bisulfite treatment is performed on the genomic DNA. Next, a primer set capable of amplifying when the CpG site is methylated and a primer set capable of amplifying when the CpG site is not methylated are prepared. Then, using the bisulfite-treated genomic DNA as a template, nucleotides containing at least one of the CpG sites are each amplified using these primer sets. Then, the DNA methylation level at the CpG site is calculated by comparing the amounts of the obtained amplification products, that is, the amount of methylated CpG site-specific amplification product with the amount of unmethylated CpG site-specific amplification product.

Furthermore, as another aspect of this fifth method, first, a bisulfite treatment is performed on the genomic DNA. Next, an oligonucleotide probe having nucleotides capable of hybridizing when the CpG site is methylated, and labeled with a fluorescent reporter dye and a fluorescent quencher dye, is prepared. In addition, an oligonucleotide probe having a nucleotide capable of hybridizing when the CpG site is not methylated, and labeled with a fluorescent reporter dye different from the above fluorescent reporter dye and a fluorescent quencher dye, is prepared. Then, the oligonucleotide probe is hybridized to the bisulfite-treated genomic DNA, and the nucleotides containing the CpG site are amplified using the genomic DNA hybridized by the oligonucleotide probe as a template. Then, the fluorescence emitted by the fluorescent reporter dye is detected by the degradation of the oligonucleotide probe associated with the amplification. The DNA methylation level at the CpG site is calculated by comparing the intensity of the fluorescence emitted by the fluorescent reporter dye specific to the methylated cytosine CpG site thus detected with the intensity of the fluorescence emitted by the fluorescent reporter dye specific to the unmethylated cytosine CpG site.

An example of the fifth method is the methylation-specific polymerase chain reaction (MS-PCR) using real-time quantitative PCR such as the MethyLight method using a TaqMan probe (registered trademark).

The sixth method is a method based on the following principles. First, a bisulfite treatment is performed on the genomic DNA. Next, a direct sequencing reaction is performed using the bisulfite-converted nucleotide containing the CpG site as a template. Then, the DNA methylation level at the CpG site is calculated by comparing the fluorescence intensity based on the determined base sequence, that is, the luminescence intensity derived from the methylated cytosine residues (luminescence intensity of cytosine) with the luminescence intensity derived from the unmethylated cytosine residues (luminescence intensity of thymine).

Furthermore, as another aspect of this sixth method, first, a bisulfite treatment is performed on the genomic DNA. Next, the bisulfite-converted nucleotides containing the CpG site are cloned by PCR reaction or the like. Then, the base sequences of the obtained multiple cloning products are each determined, and the DNA methylation level at the CpG site is calculated by comparing the number of cloning products having the methylated cytosine CpG site-specific base sequence, with the number of cloning products having the unmethylated cytosine CpG site-specific base sequence.

Examples of the sixth method include bisulfite direct sequencing and bisulfite cloning sequencing (see Kristensen LS et al., Clin Chem, 2009, 55, 1471-1483).

The seventh method is a method based on the following principles. First, a bisulfite treatment is performed on the genomic DNA. Next, a region containing the CpG site is amplified by PCR using the nucleotide containing the bisulfite-converted CpG site as a template. Next, the amplified DNA fragment is treated with a restriction enzyme that recognizes the sites different in sequence between a case where the CpG site is methylated and a case where the CpG is not methylated. Then, the DNA methylation level of the CpG site can be calculated by quantitatively analyzing the band intensity of the restriction enzyme fragment derived from the methylated CpG site and the restriction enzyme fragment derived from the unmethylated CpG site, which have been fractionated by electrophoresis.

An example of the seventh method is COBRA (analysis by the combined use of bisulfite and a restriction enzyme).

The eighth method is a method using ion exchange chromatography. For example, the eighth method is a method based on the following principles. First, a bisulfite treatment is performed on the genomic DNA. Then, it is fragmented to obtain DNA fragments containing a CpG site. The regions containing the CpG sites are amplified by PCR or the like using the obtained DNA fragments as a template. Next, the amplified DNA fragments are subjected to ion exchange chromatography to separate the DNA in which the CpG sites are methylated from the DNA in which the CpG sites are not methylated.

In the eighth method, the chain length of the PCR amplification products can be appropriately selected considering factors such as shortening the PCR amplification time, and shortening the analysis time and preserving the separation performance in the ion exchange chromatography. For example, the chain length of the PCR amplification products is preferably 500 bp or less, more preferably 300 bp or less, and further preferably 100 bp or less, on the other hand, the lower limit is 30 to 40 bp, which is the chain length of the PCR amplification products when using a primer of around 15mer which can avoid non-specific hybridization in PCR. Otherwise, it is preferable to design the primer to have a rich content of CpG sites in the PCR amplification products. For example, it is preferable that the cytosine of the CpG site be contained in 2% or more, more preferably 5% or more, with respect to the chain length of the PCR amplification products.

In the eighth method, the unmethylated cytosine residues are converted to uracil by bisulfite treatment of genomic DNA and then further converted to thymine by PCR. On the other hand, the methylated cytosine residues remain as cytosine even after a bisulfite treatment and PCR. Due to this difference in base, the fragments containing methylated cytosine (methylated fragments) and the unmethylated fragments are detected as separate peaks with different retention times in ion exchange chromatography. That is, the methylated fragments are detected as peaks having a shorter retention time than the unmethylated fragments. Therefore, it is possible to determine whether the DNA at the CpG site is methylated or not based on the retention time of the peak in ion exchange chromatography. Furthermore, when the DNA fragments to be subjected to the ion exchange chromatography contains a plurality of CpG sites, the more CpG sites are methylated, the shorter the retention time of the peak is. Therefore, the DNA methylation level at the CpG site can be calculated based on the retention time of the peak. Alternatively, it is also possible to calculate the abundance and abundance ratio of each methylated fragment and unmethylated fragment based on the area or height of the peak.

Preferably, whether the DNA of the CpG site is methylated or not, or the DNA methylation level of the CpG site is determined by comparing it with a sample (control) having a known DNA methylation level of the CpG site or by using a calibration curve prepared in advance using a sample having a known DNA methylation level. Alternatively, a retention time serving as a reference (also referred to as the reference retention time in this description) for separating the retention time of the peak of a methylated fragment having a highly methylated CpG site from the retention time of the peak of a fragment having a low methylation level using a sample having a known DNA methylation level, is determined in advance. For example, a fragment detected at a retention time earlier than the reference retention time is determined to be highly methylated DNA.

The ion exchange chromatography performed in the eighth method is preferably an anion exchange chromatography. The packing material of the column is not particularly limited as long as it is made of base material particles having a strong cationic group on the surface, but base material particles having both a strong cationic group and a weak cationic group on the packing material surface, as shown in International Publication No. WO 2012/108516, are preferable. More preferably, the base material particles are base material particles containing coated polymer particles in which a layer of a hydrophilic polymer having a strong cationic group (preferably a quaternary ammonium salt) is copolymerized on the surface of hydrophobic crosslinked polymer particles, and a weak cationic group (preferably a tertiary amino group) introduced on the surface of the coated polymer particles. The column temperature in the chromatographic analysis is preferably 30°C or more and less than 90°C.

The methods that can be suitably used as a "method for detecting the DNA methylation level" in the present invention have been exemplified above, but they are not limited thereto. In the first to eighth methods, as described above, the genomic DNA prepared from an upper urinary tract urothelial cell or a tissue is further subjected to a bisulfite treatment. Therefore, the genomic DNA used for detecting the DNA methylation level of a CpG site in the method of the present invention is preferably bisulfite-treated genomic DNA derived from an upper urinary tract urothelial cell or a tissue containing the upper urinary tract urothelial cell.

In the method of the present invention, the presence or absence of UTUC in the tested upper urinary tract urothelial cell or tissue containing an upper urinary tract urothelial cell, or the presence or absence of UTUC in the subject is determined from the detected DNA methylation levels of CpG sites. A specific index for determination can be appropriately set by a person skilled in the art according to the method for detecting the DNA methylation level.

An embodiment of the procedure for determination of the presence or absence of UTUC will be described below. In a first embodiment, first, for each DNA methylation level detection method, a receiver operating characteristic (ROC) analysis is performed for each CpG site to obtain the sensitivity (positive rate) and specificity (negative rate), then the DNA methylation level at which the sum of the sensitivity and specificity is maximum is set as an index (cutoff value).

In the first embodiment, for the CpG sites at which the methylation level is increased by the canceration of the upper urinary tract urothelial tissue (for example, the CpG sites included in the marker groups 1, 2, 4, 8, and 9 shown in Table 1), when the DNA methylation level detected in the method of the present invention is higher than the cutoff value, the DNA methylation level is considered to have exceeded the diagnostic threshold and the test cell or tissue, or subject is determined to have UTUC. But, when the detected methylation level is equal or below the cutoff value, the test cell or tissue, or subject is determined not to have UTUC. On the other hand, for the CpG sites at which the methylation level is reduced by the canceration of the upper urinary tract urothelial tissue (for example, the CpG sites included in the marker groups 3, 5, 6, and 7 shown in Table 1), when the DNA methylation level detected in the method of the present invention is lower than the cutoff value, the DNA methylation level is considered to have exceeded the diagnostic threshold, and the test cell or tissue, or subject is determined to have UTUC. But, when the detected methylation level is equal or above the cutoff value, the test cell or tissue, or subject is determined not to have UTUC.

In the first embodiment, when the methylation levels of a plurality of CpG sites are detected, the number or ratio of CpG sites at which the DNA methylation level exceeds the diagnostic threshold can be used as an index for UTUC. For example, a test cell or tissue, or a subject can be determined to have UTUC when the methylation levels exceed the diagnostic threshold in all the investigated CpG sites. Alternatively, a test cell or tissue, or a subject can be determined to have UTUC when the methylation levels of a certain percentage or more of the investigated CpG sites exceed the diagnostic threshold. Alternatively, a test cell or tissue, or a subject can be determined to have UTUC when the methylation levels exceed the diagnostic threshold in a certain proportion or more of all the investigated CpG sites. On the other hand, the test cells or tissues, or subjects that do not meet these criteria can be determined not to have UTUC.

In the second embodiment of the procedure for UTUC determination, the methylation level of a CpG site or the presence or absence of UTUC is determined by comparing the retention time of the peak obtained by the ion exchange chromatography analysis (the eighth method) on the DNA containing the target CpG site derived from the test cell or tissue, or the subject (sample), with the retention time for the DNA containing the non-methylated target CpG site (negative control) or the DNA containing the methylated target CpG site (positive control).

In the second embodiment, when a peak with a shorter retention time than that of the negative control is detected from a sample for a CpG site at which the methylation level is increased by the canceration of upper urinary tract urothelial tissue (for example, the CpG sites included in the marker groups 1, 2, 4, 8, and 9 shown in Table 1), the sample is considered to be methylated, or the test cell or tissue, or subject is determined to have UTUC. Alternatively, when a peak with a retention time similar to that of the positive control is detected from a sample, the sample is considered to be methylated, or the test cell or tissue, or subject is determined to have UTUC. On the other hand, when a peak with a longer retention time than that of the positive control is detected from a sample for a CpG site at which the methylation level is reduced by the canceration of upper urinary tract urothelial tissue (for example, the CpG sites included in the marker groups 3, 5, 6, and 7 shown in Table 1), the sample is considered to be unmethylated, or the test cell or tissue, or subject is determined to have UTUC. Alternatively, when a peak with a retention time similar to that of the negative control is detected from a sample, the sample is determined to be unmethylated, or the test cell or tissue, or subject is determined to have UTUC.

If the genomic DNA to be examined for the methylation levels of target CpG sites is DNA derived from urine, not only DNA derived from UTUC cells but also DNA derived from non-cancerous epithelial cells or cells of irrelevant lineage such as blood cells may coexist in the genomic DNA. Even in such cases, the presence or absence of DNA derived from UTUC cells can be detected by the eighth method.

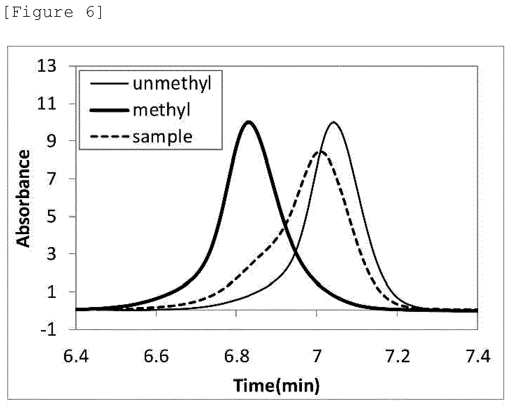

That is, if DNAs derived from a plurality of types of cells coexist in the sample DNA, a peak being bimodal, having shoulders, or being wide in shape is then exhibited in the chromatography analysis by the eighth method (for example, see Figure 6 shown later). Such an original peak can be separated into peaks derived from cells of different lineages, that is, peaks of different DNA methylation levels. For example, peaks included in an original peak can be estimated through fitting for the original peak on the assumption that a plurality of peaks in the shape of normal distribution are present. Subsequently, the methylation levels of the target CpG sites are calculated for the respective estimated peaks on the basis of the retention times. For example, the methylation level of each estimated peak can be calculated by using a calibration curve prepared on the basis of the retention times for DNAs with known methylation levels. Comparison between the methylation level of each estimated peak and the cutoff value with the same procedure as in the first embodiment described above allows determination whether the DNA derived from urine contains DNA derived from UTUC cells, or whether the subject has UTUC.

Thus, according to the present invention, UTUC can be identified with a less invasive and simpler procedure than conventional UTUC test methods such as ureteral catheterization and CT-urography. The present invention enables diagnosis of UTUC with high sensitivity and specificity with reduced physical burdens on a patient in UTUC test. In addition, the present invention enables UTUC test for patients to which it is difficult to apply conventional ureteral catheterization and CT-urography, such as patients suspected to have urethral injury, patients with contrast media allergy, and patients in renal impairment cases.

Further, the present invention provides a method for treating UTUC, which includes treating a subject determined to have UTUC by the method of the present invention. Examples of the means of treatment include surgery and chemotherapy with drugs, radiation, or the like, but are not particularly limited thereto.

Furthermore, the present invention provides a primer or a probe for determining the presence or absence of UTUC in an upper urinary tract urothelial cell, a tissue containing an upper urinary tract urothelial cell, or a subject. The primer or probe has a chain length of at least 12 bases, and hybridizes to at least one CpG site selected from the group consisting of the CpG sites shown in Table 1.

An example of the primer or probe of the present invention is a primer or probe that hybridizes to bisulfite-treated DNA containing at least one of the CpG sites shown in Table 1. Another example of the primer of the present invention is a primer that amplifies bisulfite-treated DNA containing at least one of the CpG sites shown in Table 1. Preferable examples of the DNA containing at least one of the CpG sites shown in Table 1 include DNA containing a nucleotide sequence set forth in any of SEQ ID NOs: 31 to 40.

A preferable example of the primer or probe of the present invention is a primer or probe that hybridizes to the bisulfite-treated DNA containing at least one of the CpG sites shown in Table 1, and that is constructed so as to have a complementary base to methylated cytosine or unmethylated cytosine at the 3'-terminus (for example, a primer or probe that can be used in the above-mentioned first method).

Another preferable example of the primer or probe of the present invention is a primer (sequencing primer) capable of performing an extension reaction on each base from the vicinity of the bases of the CpG sites in the bisulfite-treated DNA fragment containing at least one of the CpG sites shown in Table 1 (for example, a primer or probe that can be used in the above-mentioned third method). Another preferable example is a probe that hybridizes to nucleotides containing the CpG sites in the bisulfite-treated DNA fragment containing at least one of the CpG sites shown in Table 1 (for example, a primer or probe that can be used in the above-mentioned fourth method).

Another preferable example of the primer or probe of the present invention is a primer set capable of specifically amplifying regions containing the methylated or unmethylated CpG sites in the bisulfite-treated DNA fragment containing at least one of the CpG sites shown in Table 1 (for example, a primer set that can be used for the PCR amplification in the above-mentioned fifth method or eighth method).

The chain length of the primer or probe of the present invention may be at least 12 bases, preferably at least 15 bases, more preferably at least 20 bases, further preferably from 15 to 40 bases. Moreover, the primer or probe of the present invention may be labeled (for example, fluorescently labeled). In addition, the primer or probe of the present invention is preferably a primer or probe that can be used in any of the first to eighth methods. Moreover, the primer of the present invention is preferably a PCR primer.

Preferable examples of the primer or probe of the present invention include polynucleotides consisting of the nucleotide sequences set forth in SEQ ID NOs: 1 to 30 and the complementary strands thereof. Another preferable examples include polynucleotides consisting of nucleotide sequences having at least 90% sequence identity with the nucleotide sequences set forth in SEQ ID NOs: 1 to 30 and the complementary strands thereof. More preferable examples include a primer set consisting of a combination of the polynucleotides set forth in SEQ ID NOs: 1 and 2, or a combination of the complementary strands thereof; a primer set consisting of a combination of the polynucleotides set forth in SEQ ID NOs: 3 and 4, or a combination of the complementary strands thereof; a primer set consisting of a combination of the polynucleotides set forth in SEQ ID NOs: 5 and 6, or a combination of the complementary strands thereof; a primer set consisting of a combination of the polynucleotides set forth in SEQ ID NOs: 7 and 8, or a combination of the complementary strands thereof; a primer set consisting of a combination of the polynucleotides set forth in SEQ ID NOs: 9 and 10, or a combination of the complementary strands thereof; a primer set consisting of a combination of the polynucleotides set forth in SEQ ID NOs: 11 and 12, or a combination of the complementary strands thereof; a primer set consisting of a combination of the polynucleotides set forth in SEQ ID NOs: 13 and 14, or a combination of the complementary strands thereof; a primer set consisting of a combination of the polynucleotides set forth in SEQ ID NOs: 15 and 16, or a combination of the complementary strands thereof; a primer set consisting of a combination of the polynucleotides set forth in SEQ ID NOs: 17 and 18, or a combination of the complementary strands thereof; and a primer set consisting of a combination of the polynucleotides set forth in SEQ ID NOs: 19 and 20, or a combination of the complementary strands thereof; and a primer set which consists of a combination of primers having at least 90% sequence identity with respective polynucleotides contained in any of those primer sets and amplifies DNA containing a nucleotide sequence set forth in any of SEQ ID NOs: 31 to 40. Another more preferable examples include a probe consisting of a nucleotide sequence set forth in any of SEQ ID NOs: 21 to 30 or the complementary strand thereof; and a probe which consists of a nucleotide sequence having at least 90% sequence identity with any of SEQ ID NOs: 21 to 30 or the complementary strand thereof, and is capable of detecting any CpG site contained in a nucleotide sequence set forth in any of SEQ ID NOs: 31 to 40.

Furthermore, the present invention provides a kit for determining UTUC, which contains the primer or probe of the present invention. Preferably, the kit of the present invention is used for determining the presence or absence of UTUC in an upper urinary tract urothelial cell or a tissue containing an upper urinary tract urothelial cell, or a subject by any of the first to eighth methods.

The kit of the present invention can contain components other than the primer or probe of the present invention. Examples of such components include reagents necessary for the bisulfite treatment (for example, a sodium bisulfite solution and the like), reagents necessary for the PCR reaction (for example, deoxyribonucleotides, thermostable DNA polymerase and the like), reagents necessary for the Infinium Assay (for example, nucleotides labeled with a fluorescent substance), reagents necessary for MassARRAY (registered trademark) (for example, RNase for performing a base-specific cleavage reaction), reagents necessary for pyrosequencing (for example, ATP sulfurylase for detecting pyrophosphoric acid, adenosine-5'-phosphosulfate, luciferase, luciferin, streptavidin for separating single-stranded DNA, and the like), reagents necessary for the MS-HRM method (for example, intercalators that emit fluorescence when inserted between DNA duplexes, and the like), reagents necessary for the detection of the labels (for example, a substrate or an enzyme, positive control and negative control samples, buffer solutions used for diluting or washing samples (genomic DNA derived from tissue and the like) and the like). Moreover, the kit can also include an instructions leaflet.

Examples

Hereafter, the present invention is described in detail with examples, but the present invention is not limited to the following examples.

[Patient and Tissue Sample]

Non-cancerous upper urinary tract urothelial tissues (N) (n=31) and UTUC tissues (UTUC) (n=31) obtained from UTUC cases were used as samples for an initial cohort. Normal upper urinary tract urothelial tissues (C) (n=26) obtained from non-urothelial carcinoma cases were used as control samples. Further, urinary bladder urothelial carcinoma tissue (BUC) samples (n=14) were obtained from patients with urinary bladder urothelial carcinoma. As a validation cohort, 41 UTUC samples and 41 N samples (82 samples in total) were prepared. The tissue samples were obtained from patients who had surgery at the National Cancer Center Hospital. Written informed consent was obtained from all the patients. In addition, all the studies in the present Examples were carried out with the approval of the Ethics Committee of the National Cancer Center and Keio University School of Medicine.

The collected tissues were cryopreserved. Genomic DNA was extracted by treating the obtained fresh frozen tissue samples with phenol-chloroform followed by dialysis. 500 ng of the extracted DNA was treated with bisulfite using an EZ DNA Methylation-Gold (TM) Kit (manufactured by Zymo Research). For pyrosequencing, 400 ng of the extracted DNA was treated with bisulfite using an EpiTect Bisulfite Kit (manufactured by Qiagen).

[Example 1: Determination of UTUC identification markers]

(Infinium Assay)

The DNA methylation status at the 485,764 CpG sites was analyzed at single-CpG site resolution using Infinium (registered trademark) Human Methylation 450K BeadChip (Illumina, Inc.). The Infinium (registered trademark) Human Methylation 450K BeadChip is said to cover 99% of RefSeq genes and 96% of CpG islands. More specifically, it is said to include the promoter region, 5' untranslated region, first exon, gene body, and 3' untranslated region as target of analysis, and to cover 99% of reference sequence genes, for the purpose of comprehensively analyzing the DNA methylation status.

Specifically, a whole genome amplification treatment was performed on the bisulfite-treated DNA (see Bibikova, M. et al., Epigenomics, 2009, vol. 1, pp. 177-200). The amplified DNA fragments were hybridized to the probe on the chip, and then fluorescently labeled bases were incorporated into the hybridized DNA by a single-base extension reaction. As a result, the probe for detecting methylation hybridized to the DNA fragment containing methylated CpG and the probe for detecting unmethylation hybridized to the DNA fragment containing unmethylated CpG were each fluorescently labeled. Next, the fluorescence signal was measured using Illumina iScan System (Illumina, Inc.) according to the manufacturer's protocol. The obtained data was analyzed using GenomeStudio methylation software (Illumina, Inc.).

(Calculation of DNA Methylation Level)

At each CpG site, the relative ratio of the signal from the probe for detecting methylation to the total of signals from the probe for detecting methylation and the probe for detecting unmethylation was calculated. That is, the methylation level at each CpG site was expressed as a so-called β value (range: from 0.00 to 1.00).

(Determination of Marker CpG)

Among all of the tissue samples (26 C samples, and 31 N samples and 31 UTUC samples in the initial cohort), 893 probes with a call rate (detection P value < 0.01) of less than 90% were excluded from the analysis because they may have been designed on polymorphic sites. In addition, 11,648 probes designed on a sex chromosome and 127 probes with 10% or more of β values deleted were excluded from analysis. Eventually, DNA methylation data for 473,228 CpG sites were subjected to analysis. All statistical analyses were performed by using the statistical programs RStudio (RStudio Inc., [www.rstudio.com]) and R software (R Foundation for Statistical Computing, [www.r-project.org).

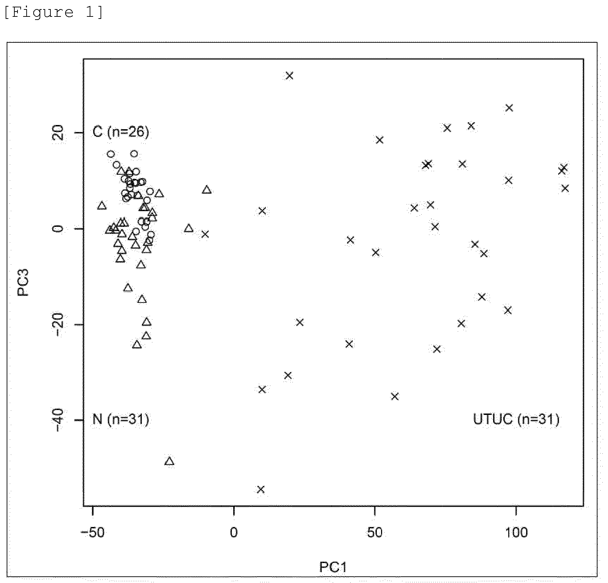

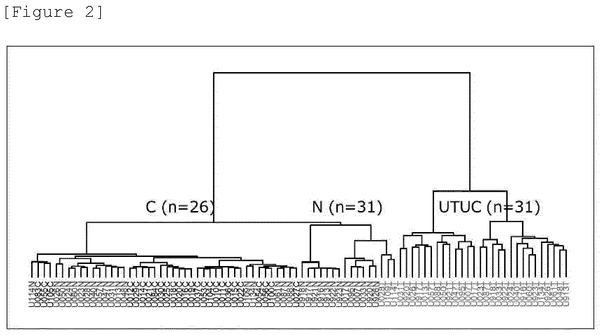

Results of the Infinium assay for the C samples (n=26), N samples (n=31) and UTUC samples (n=31) with respect to the 473,228 CpG sites were analyzed by principal component analysis and unsupervised hierarchical clustering (Euclidean distance, the Ward's method). The results of the principal component analysis found that the DNA methylation profile of the N samples exhibited a tendency different from that of the C samples, and that the UTUC samples exhibited a DNA methylation profile clearly different from those of the N samples and the C samples (Figure 1). In the hierarchical clustering, similarly, the UTUC samples exhibited a DNA methylation profile clearly different from those of the N samples and the C samples (Figure 2). These results suggested that UTUC can be distinguished from N samples and C samples on the basis of DNA methylation.

A Welch t test with Bonferroni correction was performed to detect CpG sites with significant difference in methylation level between the UTUC samples and the C samples. For each detected CpG site, a receiver operating characteristic (ROC) curve on the sensitivity and specificity for methylation level was prepared, and CpG sites having an area under the curve (AUC) of 1.0 were identified. The same analysis was performed for the UTUC samples and N samples and for the C samples and the N samples. Identified from the results were (a) 7,777 CpG sites with significant difference in methylation level between the UTUC samples and the C samples (P<0.05 and Δβ[UTUC-C]≥0.2) with the AUC of the ROC curve being 1.0, (b) 2,723 CpG sites with significant difference in methylation level between the UTUC samples and the N samples (P<0.05 and Δβ[UTUC-N]≥0.2) with the AUC of the ROC curve being 1.0, and (c) 22 CpG sites with significant difference in methylation level between the C samples and the N samples (P<0.05 and Δβ[N-C]≥0.2). Selected from them were 2,448 CpG sites which satisfied (a) and (b) but not (c), that is, exhibited statistically significant difference in methylation level between UTUC and C and between UTUC and N but did not exhibit statistically significant difference in methylation level between C and N.

From the 2,448 CpG sites, the top 100 sites with largest β value difference between UTUC and N were selected, from which 52 CpG sites positioned in a CpG island, an island shore (a region of 2000 bp adjacent to a CpG island), or an island shelf (a region of 2000 bp adjacent to an island shore) were selected by using UCSC Genome Browser ([genome.ucsc.edu/], provided by The University of California Santa Cruz (UCSC)). From the 52 sites, 21 CpG sites which exhibited relatively stable methylation levels together with nearby CpG sites (the interquartile range of the methylation levels of all the CpG sites positioned in a region of at least 1000 bp, including the target CpG site, is less than 20%) were selected by using the integrative DNA methylation database iMETHYL ([imethyl.iwate-megabank.org], provided by IWATE TOHOKU MEDICAL MEGABANK ORGANIZATION).

A Welch t test was performed for the 21 CpG sites, detecting 18 CpG sites with significant difference in methylation level between 31 UTUC samples and 14 BUC samples. These 18 CpG sites were expected to be candidate markers for identification of UTUC which allow UTUC to be distinguished not only from C and N, but also from BUC.

The DNA methylation levels of the selected candidate markers were confirmed by pyrosequencing. For 10 CpG sites for which optimization of conditions for pyrosequencing succeeded, the methylation levels of 26 C samples and those of 31 N samples and 30 UTUC samples in the initial cohort were quantified (one UTUC sample was excluded because of shortage of the amount of DNA). Table 2 shows the target CpG sites and conditions for pyrosequencing.

[Table 2]

| CpG* | PCR primer (Forward/Reverse) | SEQ ID: No | Sequencing primer | SEQ ID: No | Target sequence (each underline indicates a CpG site) | SEQ ID: No | PCR conditions |
|---|---|---|---|---|---|---|---|
| cg01921432 | 5'-GGTGGAGGTGGTGATGATGTT-3' /5'-TCAAACCACCCCATCTCCCTA-3' | 1,2 | 5'-TGGGTAGAGTTTGTTATTAGA-3' | 21 | 5'-TGCGGCTCCGGGGGCTCCA-3' | 31 | [94°C 30 sec → 56°C 30 sec → 72°C 1 min] × 50 |
| cg07197785 | 5'-GGGAAGGATTAGATAAGGTGAGGG-3' /5'-TTCCTACCCAACATCCCCTAAC-3' | 3, 4 | 5'-AGAGAGAGTTTGTTTTTGG-3' | 22 | 5'-TCGCTCCGACCCCTCGCCA-3' | 32 | [94°C 30 sec → 54°C 30 sec → 72°C 1 min] × 50 |
| cg07418387 | 5'-TGGGAAGTAAAGAGGGAAAAATG-3' /5'-TCCCATTAAAATCCATCTTCTCC-3' | 5, 6 | 5'-AGTTAATGGGGTGATTTT-3' | 23 | 5'-TCGAATTTGTGCCTCTGACCTGGAAGT-3' | 33 | [94°C 30 sec → 58°C 30 sec → 72°C 1 min] × 50 |
| cg08364561 | 5'-GGTTGGGAAGGAAGTAGAGTATAATA-3' /5'-CCCCAACCTCAAATTCTCTTA-3' | 7,8 | 5'-CCAAATAAACCACTTCTCTA-3' | 24 | 5'-GCGCTGCGGGCCGGCTACGCT-3' | 34 | [94°C 30 sec → 58°C 30 sec → 72°C 1 min] × 50 |
| cg10256242 | 5'-TTGGAAGTTTTGGGTTTAAGTGAT-3' /5'-CCCAAACTAAAAAAAAACCCATATTC-3' | 9,10 | 5'-TTGTTTAGGTTTTTTAAAGTG-3' | 25 | 5'-CCCAGCCGACGGTTCAGGTGTGTCCGTGTCT-3' | 35 | [94°C 30 sec → 57.5°C 30 sec → 72°C 1 min] × 50 |
| cg10874111 | 5'-TTAGGGTTTTTGATGAGTTAGTTTTAT-3' /5'-ACACAATCTATCCACCTCCATAA-3' | 11, 12 | 5'-TGTTTAGGGGTTAGGGAAAG-3' | 26 | 5'-AGTCAGCGCTGCACAGTCAGGCACGGGGAC-3' | 36 | [94°C 30 sec → annealing 30 sec → 72°C 1 min] × 50 The annealing temperature was decreased from 61°C by 0.1°C per cycle for 10 cycles, then retained at 60°C for 40 cycles |
| cg14302471 | 5'-TGGGTTAGGTGTTTAGGGTAGGTAG-3' /5'-CAATCTATCCACCTCCATAATCAA-3' | 13, 14 | 5'-ATAGTTAGGTAAGGGGATTT-3' | 27 | 5'-TCGCGTGTGAGTCCACGGCCTTCAGAGGGG-3' | 37 | [94°C 30 sec → 56°C 30 sec → 72°C 1 min] × 50 |
| cg14851578 | 5'-GAGGGTTGGATTGGAGGATATT-3' /5'-ACTCACCCCTCACTCAAAACA-3' | 15, 16 | 5'-GTGTGGGTTAAGTGTGAT-3' | 28 | 5'-CACGTGTGGGGTGTCTATGGCGCCCT-3' | 38 | [94°C 30 sec → 52°C 30 sec → 72°C 1 min] × 50 |

16

| CpG* | PCR primer (Forward/Reverse) | SEQ ID: No | Sequencing primer | SEQ ID: No | Target sequence (each underline indicates a CpG site) | SEQ ID: No | PCR conditions |
|---|---|---|---|---|---|---|---|
| cg15822765 | 5'-GGGGATAGAGGGTTATTTTTTGAA-3' /5'-TCTCCCCATAACCTCTTCTCTAAAA-3' | 17, 18 | 5'-TAGGTTAAAATTATTTGAAA-3' | 29 | 5'-CT<u>CG</u>CTCAG<u>CG</u>G<u>CG</u>TCGGGGCTCCA-3' | 39 | [94°C 30 sec → 52°C 30 sec → 72°C 1 min] × 50 |
| cg24035245 | 5'-TTTATAAAGGGAGTTTTGTGTGG-3' /5'-CCTAAAACAAACTCCTCCCTCATA-3' | 19, 20 | 5'-TAGTTAGATTTTAGATTGTAGGG-3' | 30 | 5'-TG<u>CG</u>CCC<u>G</u>C<u>CG</u>TTCCAGG<u>CG</u>CCAG-3' | 40 | [94°C 30 sec → 54°C 30 sec → 72°C 1 min] × 50 Final Mg$^{2+}$ concentration: 2.25 mM |

\* Each CpG site is designated by probe ID in the Infinium assay.

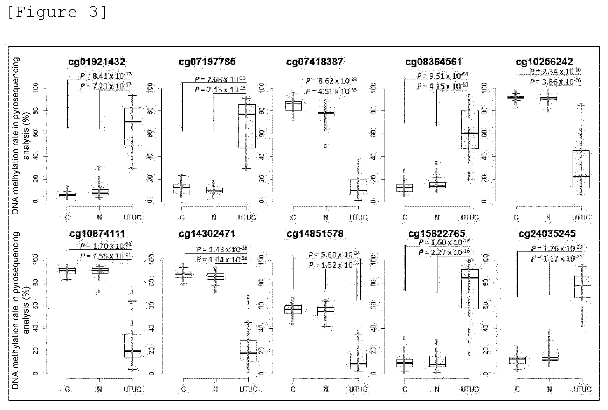

Figure 3 shows results of quantification of the methylation levels of the 10 CpG sites by pyrosequencing. No difference was found between C and N for any of the methylation levels of the CpG sites, and UTUC exhibited statistically significant difference from C and N. Statistically significant correlation between DNA methylation level in Infinium assay and DNA methylation level in pyrosequencing was found for all of the 10 CpG sites (Pearson product-moment correlation coefficient r>0.913, P<7.02×10$^{-35}$). Thus, the 10 CpG sites were confirmed to be applicable as markers for identification of UTUC.

Moreover, as shown in Table 2, CpG sites other than the 10 CpG sites detected in the Infinium assay were contained in target sequences for pyrosequencing. As predicted on the basis of the iMETHYL database, these adjacent CpG sites were predicted to be applicable as UTUC identification markers like the 10 CpG sites. Actually, CpG sites adjacent to each other were included in the 10 CpG sites detected in the Infinium assay (probe ID: cg10874111 and cg14302471). Table 3 shows the positions of the 10 CpG sites contained in target sequences for pyrosequencing and CpG sites adjacent thereto on the chromosome, and the related gene names.

[Table 3]

| CpG site | | Chromosomal allocation | | |
|---|---|---|---|---|
| Probe ID*1 | Position*2 | Chromosome No. | Position | Gene symbol*3 |
| cg01921432 | 1 | 1 | 91,182,528 | BARHL2 |
| | 2 | 1 | 91,182,534 | BARHL2 |
| cg07197785 | 1 | 1 | 111,813,685 | NA |
| | 2 | 1 | 111,813,690 | NA |
| | 3 | 1 | 111,813,698 | NA |
| cg07418387 | 1 | 1 | 240,375,464 | FMN2 |
| cg08364561 | 1 | 11 | 31,846,849 | NA |
| | 2 | 11 | 31,846,844 | NA |
| | 3 | 11 | 31,846,839 | NA |
| | 4 | 11 | 31,846,833 | NA |
| cg10256242 | 1 | 7 | 4,850,072 | RADIL |
| | 2 | 7 | 4,850,075 | RADIL |
| | 3 | 7 | 4,850,090 | RADIL |
| cg10874111 | 1 | 7 | 158,799,748 | LOC154822 |
| | 2 | 7 | 158,799,765 | LOC154822 |
| cg14302471 | 1 | 7 | 158,799,775 | LOC154822 |
| | 2 | 7 | 158,799,777 | LOC154822 |
| | 3 | 7 | 158,799,789 | LOC154822 |
| cg14851578 | 1 | 14 | 106,187,192 | NA |
| | 2 | 14 | 106,187,210 | NA |
| cg15822765 | 1 | 6 | 28,956,374 | NA |
| | 2 | 6 | 28,956,381 | NA |
| | 3 | 6 | 28,956,384 | NA |
| | 4 | 6 | 28,956,387 | NA |
| cg24035245 | 1 | 1 | 119,535,921 | NA |
| | 2 | 1 | 119,535,925 | NA |
| | 3 | 1 | 119,535,928 | NA |
| | 4 | 1 | 119,535,937 | NA |

*1 Probe ID in the Infinium assay.

*2 Each underline indicates a CpG site detected with the corresponding probe in the Infinium assay.

*3 NA: not annotated (disposed in an intergenic region)

[Example 2: Evaluation of UTUC Identification Markers]

The 10 CpG sites and CpG sites adjacent thereto were evaluated on the effectiveness as UTUC identification markers. An ROC curve on sensitivity and specificity to distinguish between the UTUC samples (n=30) and the N samples (n=31) in the initial cohort was prepared for the methylation levels of each of the 10 CpG sites and the CpG site adjacent thereto. In addition, an ROC curve was prepared in the same manner for the mean of the methylation levels of each of the 10 CpG sites and the CpG site adjacent thereto. The AUC of each of the ROC curves obtained was calculated, and the diagnostic threshold (cutoff value) was determined by using the top left method.

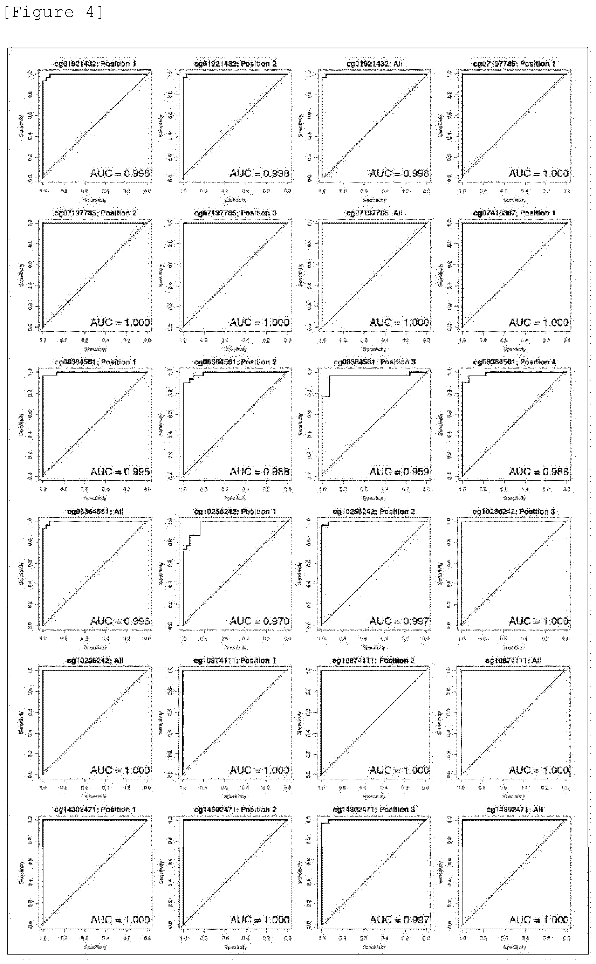

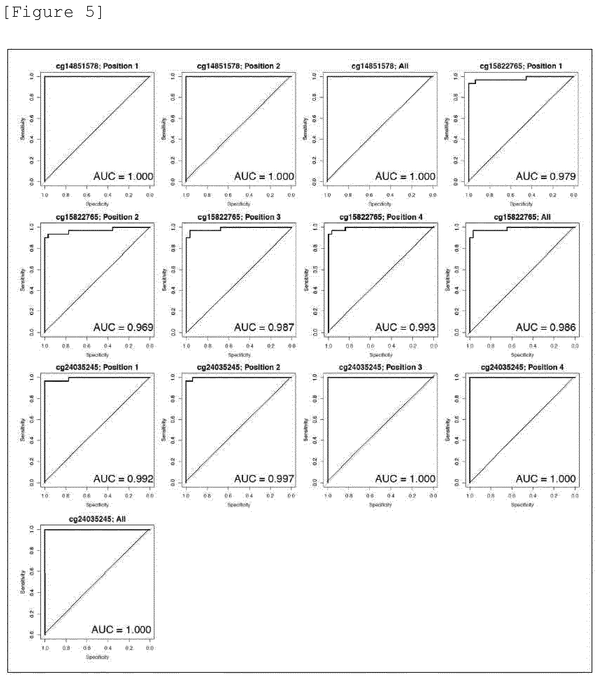

Figures 4 and 5 show the ROC curves for the CpG sites. The ROC curves for the 10 CpG sites detected in the Infinium assay each exhibited an excellent AUC value (from 0.988 to 1.000). The AUC values for the adjacent CpG sites were from 0.959 to 1.000, and the AUC values for the means for the 10 CpG sites and the respective CpG sites adjacent thereto were from 0.986 to 1.000, all being high values.

Thirty-seven markers, including the methylation levels of the CpG sites detected in the Infinium assay, the methylation levels of the respective CpG sites adjacent thereto, and the means of them (see Table 4), were set, and the sensitivity and specificity of the identification of UTUC were determined. The sensitivity of the identification of UTUC was from 86.6% to 100%, and the specificity thereof was from 93.5% to 100%.

To validate the reliability of the 37 markers, the methylation levels were examined by pyrosequencing for the UTUC samples and the N samples (each n=41) in the validation cohort, and the sensitivity and specificity of the identification of UTUC was determined on the basis of the cutoff values determined in the above. The sensitivity in the validation cohort was from 73.1% to 97.5%, and the specificity was from 87.8% to 100%. The reliability of the 37 markers was verified.

Table 4 shows the methylation status in UTUC, AUC, cutoff values, and the sensitivity and specificity of the identification of UTUC for the initial cohort and the validation cohort for the 37 markers. Table 5 shows clinicopathological parameters (sex, age, malignancy, pathological stage, and lymph node metastasis) in the initial cohort and the validation cohort. In both of the initial cohort and the validation cohort, being UTUC-positive or not as determined with the 37 markers was not correlated with the clinicopathological parameters. The 37 markers were demonstrated to allow diagnosis of UTUC irrespective of clinicopathological properties.

[Table 4]

| Marker No. | CpG site Probe ID*1 | CpG site Position*2 | DNA methylation status | AUC | Cutoff value (%) | Initial cohort Sensitivity (%) | Initial cohort Specificity (%) | Validation cohort Sensitivity (%) | Validation cohort Specificity (%) |
|---|---|---|---|---|---|---|---|---|---|
| 1 | cg01921432 | 1 | UTUC > N | 0.997 | 22.54 | 96.6 | 96.7 | 82.9 | 100 |
| 2 | | 2 | | 0.999 | 25.12 | 100 | 96.7 | 82.9 | 100 |
| 3 | | All | | 0.999 | 21.70 | 100 | 96.7 | 85.3 | 100 |
| 4 | cg07197785 | 1 | UTUC > N | 1.000 | 22.47 | 100 | 100 | 95.0 | 100 |
| 5 | | 2 | | 1.000 | 23.42 | 100 | 100 | 92.5 | 100 |
| 6 | | 3 | | 1.000 | 23.40 | 100 | 100 | 90.0 | 100 |
| 7 | | All | | 1.000 | 23.41 | 100 | 100 | 95.0 | 100 |
| 8 | cg07418387 | 1 | UTUC < N | 1.000 | 43.45 | 100 | 100 | 95.1 | 100 |
| 9 | cg08364561 | 1 | UTUC > N | 0.996 | 43.89 | 96.6 | 100 | 90.2 | 100 |
| 10 | | 2 | | 0.988 | 24.47 | 93.3 | 93.5 | 85.3 | 87.8 |
| 11 | | 3 | | 0.959 | 22.81 | 96.6 | 93.5 | 92.6 | 87.8 |
| 12 | | 4 | | 0.988 | 32.69 | 96.6 | 93.5 | 92.6 | 97.5 |
| 13 | | All | | 0.997 | 39.00 | 96.6 | 96.7 | 87.8 | 100 |
| 14 | cg10256242 | 1 | UTUC < N | 0.971 | 44.73 | 86.6 | 93.5 | 73.1 | 97.5 |
| 15 | | 2 | | 0.998 | 72.48 | 96.6 | 100 | 92.6 | 100 |
| 16 | | 3 | | 1.000 | 82.15 | 100 | 100 | 92.6 | 100 |
| 17 | | All | | 1.000 | 70.43 | 100 | 100 | 90.2 | 97.5 |
| 18 | cg10874111 | 1 | UTUC < N | 1.000 | 72.45 | 100 | 100 | 95.1 | 100 |
| 19 | | 2 | | 1.000 | 72.36 | 100 | 100 | 97.5 | 100 |
| 20 | | All | | 1.000 | 72.40 | 100 | 100 | 97.5 | 100 |
| 21 | cg14302471 | 1 | UTUC < N | 1.000 | 77.05 | 100 | 100 | 97.5 | 100 |
| 22 | | 2 | | 1.000 | 69.66 | 100 | 100 | 97.5 | 100 |
| 23 | | 3 | | 0.998 | 66.37 | 96.6 | 100 | 95.1 | 100 |
| 24 | | All | | 1.000 | 72.48 | 100 | 100 | 97.5 | 100 |
| 25 | cg14851578 | 1 | UTUC < N | 1.000 | 39.49 | 100 | 100 | 95.1 | 100 |
| 26 | | 2 | | 1.000 | 44.24 | 100 | 100 | 85.3 | 95.1 |
| 27 | | All | | 1.000 | 39.81 | 100 | 100 | 90.2 | 100 |
| 28 | cg15822765 | 1 | UTUC > N | 0.980 | 38.58 | 93.3 | 100 | 95.1 | 100 |
| 29 | | 2 | | 0.970 | 35.56 | 93.3 | 96.7 | 95.1 | 100 |

(continued)

| Marker No. | CpG site | | DNA methylation status | AUC | Cutoff value (%) | Initial cohort | | Validation cohort | |
|---|---|---|---|---|---|---|---|---|---|
| | Probe ID*1 | Position*2 | | | | Sensitivity (%) | Specificity (%) | Sensitivity (%) | Specificity (%) |
| 30 | | 3 | | 0.987 | 28.82 | 96.6 | 96.7 | 97.5 | 100 |
| 31 | | 4 | | 0.994 | 28.19 | 96.6 | 96.7 | 95.1 | 100 |
| 32 | | All | | 0.986 | 32.01 | 96.6 | 96.7 | 95.1 | 100 |
| 33 | cg24035245 | 1 | UTUC > N | 0.992 | 37.54 | 96.6 | 100 | 90.2 | 100 |
| 34 | | 2 | | 0.998 | 40.97 | 96.6 | 100 | 87.8 | 100 |
| 35 | | 3 | | 1.000 | 39.70 | 100 | 100 | 90.2 | 100 |
| 36 | | 4 | | 1.000 | 32.76 | 100 | 100 | 90.2 | 100 |
| 37 | | All | | 1.000 | 35.17 | 100 | 100 | 90.2 | 100 |

*1 Probe ID in Infinium assay.

*2 A CpG site detected with the corresponding probe in Infinium assay (underlined) and CpG sites adjacent thereto, and the mean for them (All).

[Table 5]

| Clinicopathological parameters | | Number of patients | | | |
|---|---|---|---|---|---|
| | | Patients from whom N and UTUC samples were obtained | | Patients from whom C samples were | Patients from whom BUC samples were |
| | | Initial cohort (n=31) | Validation cohort (n=41) | obtained (n=26) | obtained (n=14) |
| Gender | | | | | |
| | Male | 26 | 28 | 19 | 12 |
| | Female | 5 | 13 | 7 | 2 |
| Age (yr) | | | | | |
| | Mean (min to max) | 68.0 (45-85) | 68.0 (42-86) | 61.8 (20-82) | 67.2 (49-84) |
| Histological grade[1] | | | | | |
| | Low | 6 | 9 | | 1 |
| | High | 25 | 32 | | 13 |
| Pathological stage[2] | | | | | |
| | Ta | 1 | 6 | | 0 |
| | T1 | 11 | 11 | | 3 |
| | T2 | 6 | 3 | | 2 |
| | T3 | 13 | 20 | | 6 |
| | T4 | 0 | 1 | | 3 |
| Lymph node metastasis | | | | | |
| | Negative | 17 | 35 | | 7 |
| | Positive | 3 | 4 | | 7 |
| | Not dissected | 11 | 2 | | 0 |
| Previous treatment | | | | | |
| | BCG instillation | 0 | 1 | | 3 |
| | Neoadjuvant chemotherapy | 0 | 3 | | 0 |

[Example 3: Identification of UTUC Using Urine Specimen]

Urine can contain upper urinary tract urothelial cells and can be collected in a non-invasive manner with ease, hence being useful as a sample for identification of UTUC. In the present Example, UTUC patients were detected on the basis of the methylation levels of UTUC identification markers contained in urine samples. The methylation levels of DNA were analyzed by high-performance liquid chromatography (HPLC). A marker which undergoes an enhanced methylation level through UTUC (marker No. 37 in Table 4) was used as a UTUC identification marker.

(Patients and Sample Treatment)

Catheter urine was collected for use as urine samples from 7 UTUC patients and 55 non-UTUC individuals (urinary bladder urothelial carcinoma cases, and cases with urological consultation for a problem other than UTUC). In addition, UTUC tissues (n=10) obtained from UTUC cases, and non-UTUC tissues (n=20) consisting of urinary bladder urothelial carcinoma tissues and non-cancerous tissues obtained from UTUC cases and urinary bladder urothelial carcinoma cases were prepared as tissue samples. The urine samples and the tissue samples after collection were cryopreserved until DNA extraction.

From each urine sample, a 4 mL to 55 mL portion was taken and left to thaw to normal temperature, and DNA was extracted by using a QIAamp DNA Micro Kit (manufactured by Qiagen) in accordance with the manufacturer's protocol. DNA extraction from the tissue samples was performed with the same procedure as in Example 1. The whole volume of the extracted DNA was treated with bisulfite with the same procedure as in Example 1, and then subjected to PCR amplification using a set of the primers set forth in SEQ ID NOs: 19 and 20 under the same conditions as shown in Table 2. The PCR products obtained were purified, and subjected to HPLC. The column used in HPLC and the HPLC conditions are as follows.

(Preparation of Anion Exchange Column)

To 2000 mL of 3 wt% polyvinyl alcohol (manufactured by Nippon Synthetic Chemical Industry Co., Ltd.) aqueous solution in a reactor with a stirrer, a mixture of 200 g of tetraethylene glycol dimethacrylate (manufactured by SHIN-NAKAMURA CHEMICAL Co., Ltd.), 100 g of triethylene glycol dimethacrylate (manufactured by SHIN-NAKAMURA CHEMICAL Co., Ltd.), 100 g of glycidyl methacrylate (manufactured by Wako Pure Chemical Industries, Ltd.) and 1.0 g of benzoyl peroxide (manufactured by KISHIDA CHEMICAL Co., Ltd.) was added. The resultant was heated with stirring, and polymerized under a nitrogen atmosphere at 80°C for 1 hour. Subsequently, 100 g of trimethylammonium ethyl methacrylate chloride (manufactured by Wako Pure Chemical Industries, Ltd.) as a hydrophilic monomer having strong cationic groups was dissolved in ion-exchanged water. The resultant was added to the reactor, and polymerized similarly with stirring under a nitrogen atmosphere at 80°C for 2 hours. The resulting polymer composition was washed with water and acetone to afford coated polymer particles including a layer of a hydrophilic polymer having quaternary ammonium groups on their surfaces. The coated polymer particles obtained were subjected to measurement using a particle size distribution analyzer (AccuSizer780/manufactured by Particle Sizing Systems), and the average particle size was found to be 10 μm. In 100 mL of ion-exchanged water, 10 g of the coated polymer particles obtained was dispersed to prepare a pre-reaction slurry. Subsequently, 10 mL of N,N-dimethylaminopropylamine (manufactured by Wako Pure Chemical Industries, Ltd.), which is a reagent having weak cationic groups, was added while the slurry was stirred, and the resultant was reacted at 70°C for 4 hours. After the completion of the reaction, the supernatant was removed by using a centrifuge (manufactured by Hitachi, Ltd., "Himac CR20G"), and washing was performed with ion-exchanged water. After the washing, the supernatant was removed by using a centrifuge. This washing with ion-exchanged water was further repeated four times to afford a packing material for ion exchange chromatography having quaternary ammonium groups and tertiary amino groups on the surface of base material particles. The packing material for ion exchange chromatography obtained was packed into a stainless-steel column (column size: 4.6 mm in inner diameter × 150 mm in length) in a liquid chromatography system.

(HPLC Conditions)

System: LC-20A series (manufactured by Shimadzu Corporation)

Column: anion exchange column (prepared in the above) Eluent:

eluent A: 25 mM MES-NaOH (pH 6.0)
eluent B: 25 mM MES-NaOH (pH 6.0)
+ 2 M guanidine sulfate

Analysis time: 15 minutes
Elution method: the mixing ratio of the eluent B was linearly increased under the following gradient conditions.
0 minutes (eluent B 30%) → 10 minutes (eluent B 50%)
Specimen: PCR amplification products obtained in 3)
Flow rate: 1.0 mL/min
Detection wavelength: 260 nm
Sample injection volume: 5 μL
Column temperature: 70°C

(Calculation of Methylation Level of UTUC Identification Marker)

Figure 6 shows an exemplary chromatogram for DNA derived from a urine sample from a UTUC patient. Urine samples from UTUC patients can contain not only UTUC cells, but also non-cancerous epithelial cells, blood cells, and so on as contaminants. In analyzing DNA methylation levels through HPLC with use of a sample in which such cells of different lineages coexist, a bimodal peak, a shoulder peak as shown in Figure 6, or a wide peak is often found in the chromatogram, and a peak derived from cells of irrelevant lineage is expected to be coexist therein. In view of this, in the present Example, an original peak in each of the chromatograms obtained through HPLC for the urine samples was separated into peaks derived from cells of different lineages, that is, peaks of different DNA methylation levels, and the methylation level of the UTUC identification marker was calculated. Specifically, peaks included in each of the original peaks derived from the urine samples were estimated through fitting on the assumption that a plurality of peaks in the shape of normal distribution was present. The retention times for the estimated peaks were detected, a calibration curve was prepared from the retention time for the unmethylation peak (methylation rate: 0%) and the retention time for the methylation peak (methylation rate: 100%) each determined in the same measurement, and the methylation levels of the estimated peaks were calculated with the calibration curve. If the methylation level of an estimated peak was higher than the cutoff value of the UTUC identification marker (the cutoff value of marker No. 37 in Table 4, 35.17%), the estimated peak was regarded as a peak for DNA derived from UTUC cells. A urine sample from which a peak for DNA derived from UTUC cells was detected was determined to be a urine sample derived from a UTUC patient.

(Identification of UTUC)

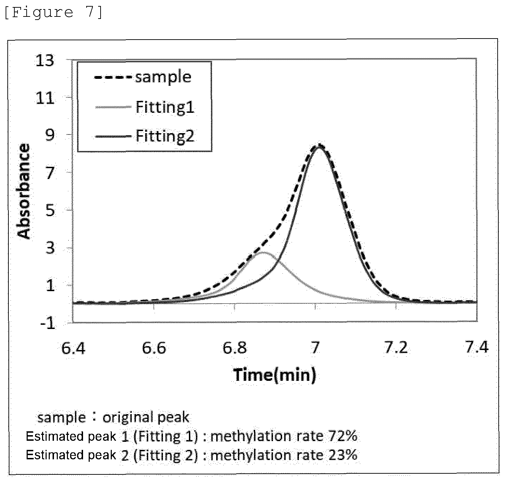

Figure 7 shows the original peak of the chromatogram shown in Figure 6, which was obtained from a urine sample from a UTUC patient, and peaks estimated as the constituent components. A peak derived from highly methylated DNA with a methylation level of 72% (estimated peak 1) was detected, and inferred to be a peak for DNA derived from UTUC cells. From the presence of the peak derived from highly methylated DNA, the patient was confirmed to be UTUC-positive.

With the same procedure, estimated peaks were detected from an original peak in each of the chromatograms for all the urine samples, and the methylation levels of the estimated peaks were calculated to determine whether the urine sample is a sample derived from a UTUC patient or not. For the tissue samples, methylation levels of DNA were calculated on the basis of original peaks. From the determination results, the sensitivity and specificity of the identification of UTUC were calculated for the urine samples, the tissue samples, and all the samples including the urine samples and tissue samples.

Table 6 shows the results. For the urine samples, the sensitivity of detection of samples derived from UTUC patients was approximately 83%, and the specificity thereof was approximately 91% (Table 6(a)). For the tissue samples, on the other hand, the sensitivity and specificity were both 100% (Table 6(b)), and the sensitivity and specificity were both approximately 94% for all the samples (Table 6(c)). These results demonstrated that identification of UTUC can be performed with high accuracy not only for tissue samples, but even for urine samples, by detecting a UTUC identification marker and referring to the methylation level thereof.

[Table 6]

| | Number of samples | | Accuracy of identification of UTUC (%) | |
|---|---|---|---|---|
| | UTUC | Non-UTUC | Sensitivity[3] | Specificity[4] |
| (a) Urine sample (n = 62) | 7 | 55[1] | 83.3 | 91.3 |
| (b) Tissue sample (n = 30) | 10 | 20[2] | 100.0 | 100.0 |
| (c) All samples (n = 92) | 17 | 75 | 93.8 | 93.9 |

[1] Urinary bladder urothelial carcinoma cases, and cases with urological consultation for a problem other than UTUC
[2] Urinary bladder urothelial carcinoma tissue, and non-cancerous tissue obtained from a surgical specimen from a UTUC case or a urinary bladder urothelial carcinoma case
[3] Proportion of UTUC samples determined to be positive
[4] Proportion of non-UTUC samples determined to be negative

## Claims

1. A method for identifying a cell or tissue having upper urinary tract urothelial carcinoma, comprising:

   detecting a DNA methylation level of at least one CpG site in genomic DNA derived from an upper urinary tract urothelial cell or a tissue containing the upper urinary tract urothelial cell, wherein the at least one CpG site is selected from the group consisting of position 158,799,748, position 158,799,765, position 158,799,775, position 158,799,777, and position 158,799,789 on chromosome 7; position 111,813,685, position 111,813,690, and position 111,813,698 on chromosome 1; position 240,375,464 on chromosome 1; position 28,956,374, position 28,956,381, position 28,956,384, and position 28,956,387 on chromosome 6; position 91,182,528 and position 91,182,534 on chromosome 1; position 119,535,921, position 119,535,925, position 119,535,928, and position 119,535,937 on chromosome 1; position 4,850,072, position 4,850,075, and position 4,850,090 on chromosome 7; position 31,846,849, position 31,846,844, position 31,846,839, and position 31,846,833 on chromosome 11; and position 106,187,192 and position 106,187,210 on chromosome 14; and
   determining whether the cell or tissue has upper urinary tract urothelial carcinoma on the basis of the detected DNA methylation level.

2. The method according to claim 1, wherein the detection of the DNA methylation level comprises detecting the DNA methylation level of the at least one CpG site using the genomic DNA treated with bisulfite.

3. The method according to claim 2, wherein the detection of the DNA methylation level is performed using a pyrosequencing method, mass spectrometry, a bead array method or ion exchange chromatography.

4. The method according to any one of claims 1 to 3, wherein the genomic DNA is DNA derived from an upper urinary tract urothelial cell contained in urine.

5. A primer or probe for identifying upper urinary tract urothelial carcinoma, which has a chain length of at least 12 bases, and is hybridized to at least one CpG site selected from the group consisting of position 158,799,748, position 158,799,765, position 158,799,775, position 158,799,777, and position 158,799,789 on chromosome 7; position 111,813,685, position 111,813,690, and position 111,813,698 on chromosome 1; position 240,375,464 on chromosome 1; position 28,956,374, position 28,956,381, position 28,956,384, and position 28,956,387 on chromosome 6; position 91,182,528 and position 91,182,534 on chromosome 1; position 119,535,921, position 119,535,925, position 119,535,928, and position 119,535,937 on chromosome 1; position 4,850,072, position 4,850,075, and position 4,850,090 on chromosome 7; position 31,846,849, position 31,846,844, position 31,846,839, and position 31,846,833 on chromosome 11; and position 106,187,192 and position 106,187,210 on chromosome 14 after being treated with bisulfite.

6. The primer or probe according to claim 5, which is hybridized to a bisulfite-treated product of DNA containing a nucleotide sequence set forth in any of SEQ ID NOs: 31 to 40.

7. The primer or probe according to claim 5 or 6, which is selected from the group consisting of a polynucleotide consisting of a nucleotide sequence set forth in any of SEQ ID NOs: 1 to 30 and a complementary strand thereof.

8. The primer or probe according to claim 7, which is

a primer set consisting of a combination of the polynucleotides set forth in SEQ ID NOs: 1 and 2, or a combination of complementary strands thereof,
a primer set consisting of a combination of the polynucleotides set forth in SEQ ID NOs: 3 and 4, or a combination of complementary strands thereof,
a primer set consisting of a combination of the polynucleotides set forth in SEQ ID NOs: 5 and 6, or a combination of complementary strands thereof,
a primer set consisting of a combination of the polynucleotides set forth in SEQ ID NOs: 7 and 8, or a combination of complementary strands thereof,
a primer set consisting of a combination of the polynucleotides set forth in SEQ ID NOs: 9 and 10, or a combination of complementary strands thereof,
a primer set consisting of a combination of the polynucleotides set forth in SEQ ID NOs: 11 and 12, or a combination of complementary strands thereof,
a primer set consisting of a combination of the polynucleotides set forth in SEQ ID NOs: 13 and 14, or a combination of complementary strands thereof,
a primer set consisting of a combination of the polynucleotides set forth in SEQ ID NOs: 15 and 16, or a combination of complementary strands thereof,
a primer set consisting of a combination of the polynucleotides set forth in SEQ ID NOs: 17 and 18, or a combination of complementary strands thereof, or
a primer set consisting of a combination of the polynucleotides set forth in SEQ ID NOs: 19 and 20, or a combination of complementary strands thereof.

9. The primer or probe according to claim 7, which is a probe consisting of a polynucleotide consisting of a nucleotide sequence set forth in any of SEQ ID NOs: 21 to 30 or a complementary strand thereof.

10. A method for identifying upper urinary tract urothelial carcinoma in a subject, comprising:

detecting a DNA methylation level of at least one CpG site in genomic DNA derived from an upper urinary tract urothelial cell or a tissue containing the upper urinary tract urothelial cell of a subject, wherein the at least one CpG site is selected from the group consisting of position 158,799,748, position 158,799,765, position 158,799,775, position 158,799,777, and position 158,799,789 on chromosome 7; position 111,813,685, position 111,813,690, and position 111,813,698 on chromosome 1; position 240,375,464 on chromosome 1; position 28,956,374, position 28,956,381, position 28,956,384, and position 28,956,387 on chromosome 6; position 91,182,528 and position 91,182,534 on chromosome 1; position 119,535,921, position 119,535,925, position 119,535,928, and position 119,535,937 on chromosome 1; position 4,850,072, position 4,850,075, and position 4,850,090 on chromosome 7; position 31,846,849, position 31,846,844, position 31,846,839, and position 31,846,833 on chromosome 11; and position 106,187,192 and position 106,187,210 on chromosome 14; and determining whether the subject has upper urinary tract urothelial carcinoma on the basis of the detected DNA methylation level.

11. The method according to claim 10, wherein the detection of the DNA methylation level comprises detecting the DNA methylation level of the at least one CpG site using the genomic DNA treated with bisulfite.

12. The method according to claim 11, wherein the detection of the DNA methylation level is performed using a pyrosequencing method, mass spectrometry, a bead array method or ion exchange chromatography.

13. The method according to any one of claims 10 to 12, wherein the genomic DNA is DNA derived from an upper urinary tract urothelial cell contained in urine.

[Figure 1]

[Figure 2]

[Figure 3]

[Figure 4]

[Figure 5]

[Figure 6]

[Figure 7]

sample : original peak
Estimated peak 1 (Fitting 1) : methylation rate 72%
Estimated peak 2 (Fitting 2) : methylation rate 23%

## INTERNATIONAL SEARCH REPORT

International application No.

PCT/JP2020/044181

### A. CLASSIFICATION OF SUBJECT MATTER

C12N 15/09(2006.01)i; C12Q 1/686(2018.01)i; C12Q 1/6869(2018.01)i; C12Q 1/6886(2018.01)i
FI: C12Q1/686 Z; C12Q1/6869 Z; C12Q1/6886 Z; C12N15/09 Z ZNA

According to International Patent Classification (IPC) or to both national classification and IPC

### B. FIELDS SEARCHED

Minimum documentation searched (classification system followed by classification symbols)
C12N15/09; C12Q1/686; C12Q1/6869; C12Q1/6886

Documentation searched other than minimum documentation to the extent that such documents are included in the fields searched

| | |
|---|---|
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

Electronic data base consulted during the international search (name of data base and, where practicable, search terms used)
JSTPlus/JMEDPlus/JST7580 (JDreamIII); CAplus/MEDLINE/EMBASE/BIOSIS/WPIDS (STN); GenBank/EMBL/DDBJ/GeneSeq

### C. DOCUMENTS CONSIDERED TO BE RELEVANT

| Category* | Citation of document, with indication, where appropriate, of the relevant passages | Relevant to claim No. |
|---|---|---|
| X<br>A | WO 2019/199696 A1 (SINGLERA GENOMICS, INC.) 17 October 2019 (2019-10-17) fig. 5(table 1), 13/204, SEQ ID NO: 761, 771 | 5-6<br>1-4, 7-13 |
| Y | 藤本真央ほか，ゲノム網羅的 DNA メチル化解析に基づく上部尿路上皮がん診断指標開発，日本病理学会会誌，15 April 2019, vol. 108, no. 1, p. 401, P2-58, lines 4-14, non-official translation (FUJIMOTO, Mao et al., "Development of diagnostic indicators for upper urothelial carcinoma based on genome-wide DNA methylation analysis", Proceedings of the Japanese Society of Pathology) | 1-13 |
| Y | WO 2019/181941 A1 (KEIO GIJUKU) 26 September 2019 (2019-09-26) claims | 1-13 |

☒ Further documents are listed in the continuation of Box C.  ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 19 January 2021 (19.01.2021) | 26 January 2021 (26.01.2021) |

| Name and mailing address of the ISA/<br>Japan Patent Office<br>3-4-3, Kasumigaseki, Chiyoda-ku,<br>Tokyo 100-8915, Japan | Authorized officer |
|---|---|
| | Telephone No. |

Form PCT/ISA/210 (second sheet) (January 2015)

**INTERNATIONAL SEARCH REPORT**

International application No.

PCT/JP2020/044181

C (Continuation). DOCUMENTS CONSIDERED TO BE RELEVANT

| Category* | Citation of document, with indication, where appropriate, of the relevant passages | Relevant to claim No. |
|---|---|---|
| A | HASSLER, M. R. et al., "Genome-wide DNA methylation profiling of upper tract urothelial carcinoma", European Urology Supplements, 16 March 2018, vol. 17, no. 2, e1612, lines 9-13 | 1-13 |

Form PCT/ISA/210 (continuation of second sheet) (January 2015)

**INTERNATIONAL SEARCH REPORT**

Information on patent family members

International application No.

PCT/JP2020/044181

| Patent Documents referred in the Report | Publication Date | Patent Family | Publication Date |
|---|---|---|---|
| WO 2019/199696 A1 | 17 Oct. 2019 | (Family: none) | |
| WO 2019/181941 A1 | 26 Sep. 2019 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013168644 A
- WO 2015129916 A
- WO 2017038983 A
- WO 2012108516 A

**Non-patent literature cited in the description**

- *Carcinogenesis,* 2011, vol. 32, 462-9
- *Cancer Sci,* 2010, vol. 101, 231-40
- *J Urology,* 2005, vol. 173, 243-6
- *Curr Urol Rep,* 2018, vol. 19, 102
- *Epigenomics,* 2016, vol. 8, 1415-1428
- *Nature,* 2014, vol. 507, 315-22
- *Carcinogenesis,* 2019, 112
- *Int J Mol Sci,* 2019, vol. 20, E2657
- *Eur Urol,* 2017, vol. 72, 641-649
- **JURINKE C et al.** *Mutat Res,* 2005, vol. 573, 83-95
- *Anal. Biochem.,* 2000, vol. 10, 103-110
- **WOJDACZ TK et al.** *Nat Protoc.,* 2008, vol. 3, 1903-1908
- **KRISTENSEN LS et al.** *Clin Chem,* 2009, vol. 55, 1471-1483
- **BIBIKOVA, M. et al.** *Epigenomics,* 2009, vol. 1, 177-200

SEQUENCE LISTING

<110>  KEIO UNIVERSITY
       SEKISUI MEDICAL CO., LTD.

<120>  Method of determinating upper urinary tract urothelial carcinoma

<130>  DC0124

<150>  JP 2019-214647
<151>  2019/11/27

<160>  40

<170>  PatentIn version 3.5

<210>  1
<211>  21
<212>  DNA
<213>  Artificial sequence

<220>
<223>  Oligonucleotide primer

<400>  1
ggtggaggtg gtgatgatgt t                                          21


<210>  2
<211>  21
<212>  DNA
<213>  Artificial sequence

<220>
<223>  Oligonucleotide primer

<400>  2
tcaaaccacc ccatctccct a                                          21


<210>  3
<211>  24
<212>  DNA
<213>  Artificial sequence

<220>
<223>  Oligonucleotide primer

<400>  3
gggaaggatt agataaggtg aggg                                       24


<210>  4
<211>  22
<212>  DNA
<213>  Artificial sequence

<220>
<223>  Oligonucleotide primer

<400>  4
ttcctaccca acatccccta ac                                         22

```
<210>    5
<211>    23
<212>    DNA
<213>    Artificial sequence

<220>
<223>    Oligonucleotide primer

<400>    5
tgggaagtaa agagggaaaa atg                                              23


<210>    6
<211>    23
<212>    DNA
<213>    Artificial sequence

<220>
<223>    Oligonucleotide primer

<400>    6
tcccattaaa atccatcttc tcc                                              23


<210>    7
<211>    26
<212>    DNA
<213>    Artificial sequence

<220>
<223>    Oligonucleotide primer

<400>    7
ggttgggaag gaagtagagt ataata                                          26


<210>    8
<211>    21
<212>    DNA
<213>    Artificial sequence

<220>
<223>    Oligonucleotide primer

<400>    8
ccccaacctc aaattctctt a                                               21


<210>    9
<211>    24
<212>    DNA
<213>    Artificial sequence

<220>
<223>    Oligonucleotide primer

<400>    9
ttggaagttt tgggtttaag tgat                                            24


<210>    10
<211>    25
```

```
<212>   DNA
<213>   Artificial sequence


<220>
<223>   Oligonucleotide primer


<400>   10
cccaaactaa aaaaaaccca tattc                                           25



<210>   11
<211>   27
<212>   DNA
<213>   Artificial sequence


<220>
<223>   Oligonucleotide primer


<400>   11
ttagggtttt tgatgagtta gttttat                                         27



<210>   12
<211>   23
<212>   DNA
<213>   Artificial sequence


<220>
<223>   Oligonucleotide primer


<400>   12
acacaatcta tccacctcca taa                                             23



<210>   13
<211>   25
<212>   DNA
<213>   Artificial sequence


<220>
<223>   Oligonucleotide primer


<400>   13
tgggttaggt gtttagggta ggtag                                           25



<210>   14
<211>   24
<212>   DNA
<213>   Artificial sequence


<220>
<223>   Oligonucleotide primer


<400>   14
caatctatcc acctccataa tcaa                                            24



<210>   15
<211>   22
<212>   DNA
<213>   Artificial sequence
```

```
<220>
<223>   Oligonucleotide primer

<400>   15
gagggttgga ttggaggata tt                                              22


<210>   16
<211>   21
<212>   DNA
<213>   Artificial sequence

<220>
<223>   Oligonucleotide primer

<400>   16
actcacccct cactcaaaac a                                               21


<210>   17
<211>   24
<212>   DNA
<213>   Artificial sequence

<220>
<223>   Oligonucleotide primer

<400>   17
ggggatagag ggttattttt tgaa                                            24


<210>   18
<211>   25
<212>   DNA
<213>   Artificial sequence

<220>
<223>   Oligonucleotide primer

<400>   18
tctccccata acctcttctc taaaa                                           25


<210>   19
<211>   23
<212>   DNA
<213>   Artificial sequence

<220>
<223>   Oligonucleotide primer

<400>   19
tttataaagg gagttttgtg tgg                                             23


<210>   20
<211>   24
<212>   DNA
<213>   Artificial sequence

<220>
<223>   Oligonucleotide primer
```

```
<400>  20
cctaaaacaa actcctccct cata                                              24


<210>  21
<211>  21
<212>  DNA
<213>  Artificial sequence

<220>
<223>  Oligonucleotide primer

<400>  21
tgggtagagt ttgttattag a                                                 21


<210>  22
<211>  19
<212>  DNA
<213>  Artificial sequence

<220>
<223>  Oligonucleotide primer

<400>  22
agagagagtt tgtttttgg                                                    19


<210>  23
<211>  18
<212>  DNA
<213>  Artificial sequence

<220>
<223>  Oligonucleotide primer

<400>  23
agttaatggg gtgatttt                                                     18


<210>  24
<211>  20
<212>  DNA
<213>  Artificial sequence

<220>
<223>  Oligonucleotide primer

<400>  24
ccaaataaac cacttctcta                                                   20


<210>  25
<211>  21
<212>  DNA
<213>  Artificial sequence

<220>
<223>  Oligonucleotide primer

<400>  25
ttgtttaggt tttttaaagt g                                                 21
```

```
<210>  26
<211>  20
<212>  DNA
<213>  Artificial sequence

<220>
<223>  Oligonucleotide primer

<400>  26
tgtttagggg ttagggaaag                                                       20


<210>  27
<211>  20
<212>  DNA
<213>  Artificial sequence

<220>
<223>  Oligonucleotide primer

<400>  27
atagttaggt aaggggattt                                                       20


<210>  28
<211>  18
<212>  DNA
<213>  Artificial sequence

<220>
<223>  Oligonucleotide primer

<400>  28
gtgtgggtta agtgtgat                                                         18


<210>  29
<211>  20
<212>  DNA
<213>  Artificial sequence

<220>
<223>  Oligonucleotide primer

<400>  29
taggttaaaa ttatttgaaa                                                       20


<210>  30
<211>  23
<212>  DNA
<213>  Artificial sequence

<220>
<223>  Oligonucleotide primer

<400>  30
tagttagatt ttagattgta ggg                                                   23


<210>  31
<211>  19
```

<212>    DNA
<213>    Homo sapiens

<400>    31
tgcggctccg ggggctcca                                                    19


<210>    32
<211>    19
<212>    DNA
<213>    Homo sapiens

<400>    32
tcgctccgac ccctcgcca                                                    19


<210>    33
<211>    27
<212>    DNA
<213>    Homo sapiens

<400>    33
tcgaatttgt gcctctgacc tggaagt                                           27


<210>    34
<211>    21
<212>    DNA
<213>    Homo sapiens

<400>    34
gcgctgcggg ccggctacgc t                                                 21


<210>    35
<211>    31
<212>    DNA
<213>    Homo sapiens

<400>    35
cccagccgac ggttcaggtg tgtccgtgtc t                                      31


<210>    36
<211>    30
<212>    DNA
<213>    Homo sapiens

<400>    36
agtcagcgct gcacagtcag gcacggggac                                        30


<210>    37
<211>    30
<212>    DNA
<213>    Homo sapiens

<400>    37
tcgcgtgtga gtccacggcc ttcagagggg                                        30


<210>    38
<211>    26

```
<212>  DNA
<213>  Homo sapiens

<400>  38
cacgtgtggg gtgtctatgg cgccct                                          26


<210>  39
<211>  25
<212>  DNA
<213>  Homo sapiens

<400>  39
ctcgctcagc ggcgtcgggg ctcca                                           25


<210>  40
<211>  24
<212>  DNA
<213>  Homo sapiens

<400>  40
tgcgcccgcc gttccaggcg ccag                                            24
```